(19) Europäisches Patentamt / European Patent Office / Office européen des brevets

(11) **EP 4 610 255 A1**

(12) **EUROPEAN PATENT APPLICATION**
published in accordance with Art. 153(4) EPC

(43) Date of publication:
**03.09.2025   Bulletin 2025/36**

(21) Application number: **23881944.5**

(22) Date of filing: **27.10.2023**

(51) International Patent Classification (IPC):
**C07D 301/19** *(2006.01)*     **C07D 303/04** *(2006.01)*
**B01J 8/04** *(2006.01)*

(52) Cooperative Patent Classification (CPC):
**B01J 8/00; B01J 8/04; C07D 301/19; C07D 303/04**

(86) International application number:
**PCT/CN2023/127003**

(87) International publication number:
**WO 2024/088369 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **27.10.2022   CN 202211330375**

(71) Applicants:
• **China Petroleum & Chemical Corporation
Beijing 100728 (CN)**
• **Sinopec (Shangai) Research Institute of
Petrochemical Technology Co., Ltd.
Shanghai 201208 (CN)**

(72) Inventors:
• **LI, Mujin
Shanghai 201208 (CN)**
• **GAO, Rui
Shanghai 201208 (CN)**
• **SHI, Depan
Shanghai 201208 (CN)**
• **ZHAI, Zhongxi
Shanghai 201208 (CN)**
• **ZHAO, Peng
Shanghai 201208 (CN)**

(74) Representative: **karo IP
Patentanwälte PartG mbB
Steinstraße 16-18
40212 Düsseldorf (DE)**

(54) **ALKYLENE OXIDE PREPARATION METHOD AND SYSTEM**

(57)     The present invention discloses a method and system for preparing alkylene oxide. The method comprises feeding a feed comprising an olefin, an alkylbenzene hydroperoxide and an alkylbenzene into a reaction zone comprising at least two catalyst beds connected in series to carry out an epoxidation reaction to obtain a reaction product comprising an alkylene oxide, wherein when the logically first stage catalyst bed is deactivated, it is switched out and a fresh catalyst bed or a regenerated catalyst bed is switched in; wherein the inlet temperature of the feed entering the switched-in fresh catalyst bed or regenerated catalyst bed satisfies the following formula (1) such that the temperature of the discharge leaving the switched-in fresh catalyst bed or regenerated catalyst bed is lower than or equal to 130 °C:

$$40^{\circ}\text{C} \le T_N^{inlet} \le 130^{\circ}\text{C} + \sum_{i=1}^{N-1}(T_i^{outlet} - T_i^{inlet}) - \frac{1370}{a_1 + 1.38b_1} \quad (1).$$

The system is used for preparing alkylene oxide, and the system comprises a temperature controller, which controls the inlet temperature of the feed to the newly switched-in fresh catalyst bed or regenerated catalyst bed such that the inlet temperature satisfies the formula (1).

**(Cont. next page)**

EP 4 610 255 A1

Fig. 1

**Description**

**Technical Field**

The invention belongs to the field of epoxidation reaction, and particularly relates to an epoxidation method and a system for carry outing epoxidation.

**Background Arts**

Similar to oxidation reaction, epoxidation reaction is a highly exothermic reaction. For example, the liquid-phase catalytic reaction for preparing propylene oxide from cumene hydroperoxide (CHP) and propylene is a highly exothermic reaction. In a highly exothermic reaction, if the reactants and/or products are very sensitive to temperature (i.e., heat-sensitive substances), additional heat removal means or temperature control schemes are required in the reaction system to maintain the reactor operating within a reasonable reaction temperature. If the temperature control of the reaction system is inappropriate, the selectivity of the reaction products will decrease, and in severe cases, reaction temperature runaway occurs, leading to safety accident.

The effect of reactor inlet temperature on peroxide conversion rate in an adiabatic fixed bed epoxidation reactor can be found in patent CN1692106A, for example. For an adiabatic fixed bed reactor, in order to prevent temperature runaway, the conversion rate of peroxide can only be stably controlled below 20% under normal conditions. Patent CN1692106A discloses a method for performing a highly exothermic reaction, wherein multiple independent reaction zones are connected in series, and propylene is fed in a single stage and the organic peroxide is fed in multiple stages in parallel, so that the peroxide concentration in each reaction zone feed is below 8wt%, thereby stabilizing the reaction temperature. CN105294606A discloses a reaction method for preparing propylene oxide from ethylbenzene hydroperoxide and propylene, wherein the reaction system comprises at least two adiabatic fixed bed catalytic reactors connected in series, wherein propylene and a raw material containing ethylbenzene hydroperoxide are mixed and passed into a first stage reactor, and the concentration of ethylbenzene hydroperoxide and the temperature of the inlet material at the inlet of the second stage reactor are adjusted by circulating material from the outlet of the second stage reactor. CN110787739A discloses an installation and method for reacting an alkylbenzene peroxide with a lower carbon olefin to generate alkylene oxide. The reaction system includes an isothermal bed reactor and an optional adiabatic bed reactor, wherein the isothermal bed reactor is provided with heat exchange inlet and outlet pipelines to control the reaction temperature rise. Since the epoxidation reaction is highly exothermic, the isothermal bed reactor will form a large temperature gradient in the axial and radial directions of the reactor since the heat removal capacity of the isothermal bed reactor is limited by heat transfer, which may cause part of the catalyst to be in a state of high temperature and severe side reactions.

Since organic peroxides are easily decomposed by heat, in order to ensure the safety and economy of the subsequent product separation process, a complete conversion of the organic peroxide in the epoxidation reaction zone is important and necessary. Patent CN1325484C discloses an epoxidation reaction system using a fixed bed reactor equipped with a solid epoxidation catalyst, which uses multiple fixed bed reactors connected in series equipped with fresh catalyst and partially deactivated catalyst, wherein the olefin is fed sequentially into the reactors equipped with the fresh catalyst, and the organic peroxide is fed in parallel into the reactors equipped with the fresh catalyst, wherein the effluent of the reactor equipped with the fresh catalyst must be passed into at least one fixed bed reactor equipped with the partially deactivated catalyst such that the organic peroxide is completely converted. Patent CN105272947A discloses a method for continuous production of epichlorohydrin, wherein a plurality of reactors connected in series are arranged in the reaction zone, propylene enters the reactors sequentially and the organic peroxide enters each reactor in parallel, wherein the temperature of the last reactor needs to be higher than the temperature of the last but one reactor, thereby ensuring that the conversion rate of the organic peroxide in the last reactor is 100%.

The catalyst for epoxidation reaction deactivates quickly, and it is repeatedly necessary to switch out the deactivated catalyst from the reaction system and replace it with a fresh catalyst. When the epoxidation reactor is switched back into the system after replacing with the fresh catalyst, the organic hydrocarbon hydroperoxide entering the reactor can be completely converted due to the high activity of the fresh catalyst; if the inlet temperature of the epoxidation reactor is too high at this moment, the temperature rise of the epoxidation reactor will exceed the normal control value, the side reactions will increase, and the product selectivity will be greatly reduced.

**Description of the invention**

In order to overcome at least one of the above problems existing in the prior art, the present invention provides an epoxidation method (i.e., a method for preparing alkylene oxide), a method for controlling the temperature of the discharge leaving a newly switched-in fresh catalyst bed or regenerated catalyst bed in a method for preparing alkylene oxide, and a system for preparing alkylene oxide, wherein the methods and system can control the inlet temperature of the feed to the newly switched-in catalyst bed according to parameters such as the temperature rise of catalyst bed and the molar ratios between components in the feedstock, thereby controlling the temperature of the discharge leaving the newly switched-in catalyst bed. The system and methods can prevent the reaction from being out of control due to the reaction heat, and can improve the conversion rate of the reactant cumene hydroperoxide and the selectivity to the target alkylene oxide, such as propylene oxide.

The inventors of the present invention have surprisingly found through a large number of experiments that, for the method for preparing alkylene oxide of the present invention, when the reaction temperature is higher than 130 °C, the selectivity to the target alkylene oxide is significantly reduced and the impurity content in the outlet product is significantly increased. Therefore, one of the objects of the present invention is to provide a method for preparing alkylene oxide (epoxidation method), wherein the temperature of the discharge of the newly switched-in fresh catalyst bed or regenerated catalyst bed can be controlled to be lower than or equal to 130 °C.

One aspect of the present invention relates to a method for preparing alkylene oxide, including feeding a feed comprising an olefin, an alkylbenzene hydroperoxide and an alkylbenzene into an epoxidation reaction zone comprising at least two catalyst beds connected in series to carry out an epoxidation reaction to obtain a reaction product comprising an alkylene oxide, wherein the feed enters the logically first stage catalyst bed of the at least two catalyst beds connected in series and passes sequentially through the at least two catalyst beds connected in series, wherein when a catalyst bed is deactivated, for example when the logically first stage catalyst bed is deactivated, the deactivated catalyst bed is switched out and a fresh catalyst bed or a regenerated catalyst bed is switched in;

wherein the inlet temperature of the feed entering the switched-in fresh catalyst bed or regenerated catalyst bed satisfies the following formula (1) such that the temperature of the discharge leaving the switched-in fresh catalyst bed or regenerated catalyst bed is lower than or equal to 130 °C:

$$40°C \leq T_N^{inlet} \leq 130°C + \sum_{i=1}^{N-1}(T_i^{outlet} - T_i^{inlet}) - \frac{1370}{a_1 + 1.38b_1} \qquad (1)$$

in formula (1), $T_N^{inlet}$ represents the inlet temperature of the fresh catalyst bed or the regenerated catalyst bed, N represents that the switched-in fresh catalyst bed or regenerated catalyst bed is located at the logically N-th stage in the catalyst beds connected in series, $T_i^{outlet}$ represents the outlet temperature of the catalyst bed at the logically i-th stage in the catalyst beds connected in series after the fresh catalyst bed or the regenerated catalyst bed is switched in, $T_i^{inlet}$ represents the inlet temperature of the catalyst bed at the logically i-th stage, $\sum_{i=1}^{N-1}(T_i^{outlet} - T_i^{inlet})$ represents the sum of the temperature rises of all catalyst beds upstreaming the switched-in fresh catalyst bed or regenerated catalyst bed, $a_1$ represents the molar ratio of the olefin to the alkylbenzene hydroperoxide in the feed, and $b_1$ represents the molar ratio of the alkylbenzene to the alkylbenzene hydroperoxide in the feed.

Another aspect of the present invention relates to a method for controlling the temperature of the discharge leaving a newly switched-in fresh catalyst bed or regenerated catalyst bed in a method for preparing alkylene oxide to be lower than or equal to 130 °C, the method comprises feeding a feed comprising an olefin, an alkylbenzene hydroperoxide and an alkylbenzene into an epoxidation reaction zone comprising at least two catalyst beds connected in series to carry out an epoxidation reaction to obtain a reaction product comprising an alkylene oxide, wherein the feed enters the logically first stage catalyst bed of the at least two catalyst beds connected in series and passes sequentially through the at least two catalyst beds connected in series, wherein when the logically first stage catalyst bed is deactivated, it is switched out and a fresh catalyst bed or a regenerated catalyst bed is switched in; wherein the method comprises making the inlet temperature of the feed entering the switched-in fresh catalyst bed or regenerated catalyst bed satisfy the following formula (1):

$$40°C \leq T_N^{inlet} \leq 130°C + \sum_{i=1}^{N-1}(T_i^{outlet} - T_i^{inlet}) - \frac{1370}{a_1 + 1.38b_1} \qquad (1);$$

in formula (1), $T_N^{inlet}$ represents the inlet temperature of the fresh catalyst bed or the regenerated catalyst bed, N represents that the switched-in fresh catalyst bed or regenerated catalyst bed is located at the logically N-th stage in the catalyst beds connected in series, $T_i^{outlet}$ represents the outlet temperature of the catalyst bed at the logically i-th stage in the catalyst beds connected in series after the fresh catalyst bed or the regenerated catalyst bed is switched in, $T_i^{inlet}$ represents the inlet temperature of the catalyst bed at the logically i-th stage, $\sum_{i=1}^{N-1}(T_i^{outlet}-T_i^{inlet})$ represents the sum of the temperature rises of all catalyst beds upstreaming the switched-in fresh catalyst bed or regenerated catalyst bed, $a_1$ represents the molar ratio of the olefin to the alkylbenzene hydroperoxide in the feed, and $b_1$ represents the molar ratio of the alkylbenzene to the alkylbenzene hydroperoxide in the feed.

As understood by those skilled in the art, in the method for preparing alkylene oxide from an olefin and an alkylbenzene hydroperoxide, the catalyst bed at the logically first stage of the catalyst beds connected in series (i.e., the first bed) has a faster catalyst deactivation rate in this catalyst bed than the catalyst deactivation rate in other catalyst beds because the content of alkylbenzene hydroperoxide entering this bed is higher; after the catalyst is deactivated, it is necessary to switch the deactivated catalyst bed out of the system and switch in a fresh catalyst bed or a regenerated catalyst bed.

When a fresh catalyst bed or a regenerated catalyst bed is switched in, the alkylbenzene hydroperoxide entering this bed can be completely converted due to the higher activity of the fresh catalyst or the regenerated catalyst. If the inlet temperature of the feed entering this fresh catalyst bed or regenerated catalyst bed is too high at this moment, the temperature rise of the fresh catalyst bed or the regenerated catalyst bed will exceed the normal control value, resulting in an increase in side reactions and a significant decrease in product selectivity.

It is surprisingly found by the present application that by controlling the inlet temperature of the feed entering the newly switched-in fresh catalyst bed or regenerated catalyst bed to satisfy the following formula (1):

$$40℃ \leq T_N^{inlet} \leq 130℃ + \sum_{i=1}^{N-1}(T_i^{outlet}-T_i^{inlet}) - \frac{1370}{a_1+1.38b_1} \qquad (1);$$

preferably, by controlling the inlet temperature of the feed entering the newly switched-in fresh catalyst bed or regenerated catalyst bed to satisfy the following formula (1a) :

$$90℃ + \sum_{i=1}^{N-1}(T_i^{outlet}-T_i^{inlet}) - \frac{1370}{a_1+1.38b_1} \leq T_N^{inlet} \leq 130℃ + \sum_{i=1}^{N-1}(T_i^{outlet}-T_i^{inlet}) - \frac{1370}{a_1+1.38b_1} \qquad (1a)$$

preferably, to satisfy the following formula (1b):

$$100℃ + \sum_{i=1}^{N-1}(T_i^{outlet}-T_i^{inlet}) - \frac{1370}{a_1+1.38b_1} \leq T_N^{inlet} \leq 130℃ + \sum_{i=1}^{N-1}(T_i^{outlet}-T_i^{inlet}) - \frac{1370}{a_1+1.38b_1} \qquad (1b)$$

preferably, to satisfy the following formula (1c):

$$110℃ + \sum_{i=1}^{N-1}(T_i^{outlet}-T_i^{inlet}) - \frac{1370}{a_1+1.38b_1} \leq T_N^{inlet} \leq 130℃ + \sum_{i=1}^{N-1}(T_i^{outlet}-T_i^{inlet}) - \frac{1370}{a_1+1.38b_1} \qquad (1c)$$

more preferably, by controlling the inlet temperature of the feed entering the newly switched-in fresh catalyst bed or regenerated catalyst bed to satisfy the following formula (2):

$$120℃ + \sum_{i=1}^{N-1}(T_i^{outlet}-T_i^{inlet}) - \frac{1370}{a_1+1.38b_1} \leq T_N^{inlet} \leq 130℃ + \sum_{i=1}^{N-1}(T_i^{outlet}-T_i^{inlet}) - \frac{1370}{a_1+1.38b_1} \qquad (2)$$

the temperature of the discharge leaving the newly switched-in fresh catalyst bed or regenerated catalyst bed can be effectively controlled to be lower than or equal to 130 °C.

In formulae (1), (1a), (1b), (1c) and (2), $T_N^{inlet}$ represents the inlet temperature of the feed entering the newly switched-in fresh catalyst bed or regenerated catalyst bed; that is, the temperature of the feed entering the bed.

In formulae (1), (1a), (1b), (1c) and (2), N represents the stage of the newly switched-in fresh catalyst bed or regenerated catalyst bed in all the catalyst beds connected in series in the epoxidation reaction zone. That is, after the fresh catalyst bed or the regenerated catalyst bed is switched in the epoxidation reaction zone, all the catalyst beds connected in series in the epoxidation reaction zone are numbered as 1, 2,..., N-1, N, N+1,... in sequence according to the logical stages (i.e., the sequence in series connection), wherein the newly switched-in fresh catalyst bed or regenerated catalyst bed is numbered N (i.e., the N-th bed in the catalyst bed sequence connected in series). It should be noted that the above numbering sequence "1, 2,..., N-1, N, N+1,..." is merely exemplary, and in fact N can be 1 (i.e., the first bed in the catalyst bed sequence connected in series(the bed at the logically first stage)), can be 2 (i.e., the second bed in the catalyst bed sequence connected in series), can be 3, 4, 5, 6, etc. Alternatively, the total number of all catalyst beds connected in series in the epoxidation reaction zone can be N, i.e., the newly switched-in fresh catalyst bed or regenerated catalyst bed is the last bed (i.e., the logically last stage).

In formulae (1), (1a), (1b), (1c) and (2), $T_i^{outlet}$ represents the outlet temperature of the discharge of the catalyst bed (i.e., the temperature of the discharge leaving the catalyst bed) at logically i-th stage of the catalyst beds connected in series (i.e., the i-th bed in the catalyst bed sequence connected in series) after the fresh catalyst bed or the regenerated catalyst bed is switched in. In formulae (1), (1a), (1b), (1c) and (2), $T_i^{inlet}$ represents the inlet temperature of the feed to the catalyst bed at the logically i-th stage (i.e., the temperature of the feed entering the bed).

In formulae (1), (1a), (1b), (1c) and (2), $\sum_{i=1}^{N-1}(T_i^{outlet} - T_i^{inlet})$ represents the sum of the temperature rises of all catalyst beds upstreaming the switched-in fresh catalyst bed or regenerated catalyst bed in the sequence of all catalyst beds connected in series in the epoxidation reaction zone.

In formulae (1), (1a), (1b), (1c) and (2), $a_1$ represents the molar ratio of the olefin to the alkylbenzene hydroperoxide in the feed, and $b_1$ represents the molar ratio of the alkylbenzene to the alkylbenzene hydroperoxide in the feed. Please note that $a_1$ represents the molar ratio of the olefin to the alkylbenzene hydroperoxide in the feed entering the logically first stage catalyst bed (i.e., the first bed) of the catalyst bed sequence connected in series, and $b_1$ represents the molar ratio of the alkylbenzene to the alkylbenzene hydroperoxide in the feed entering the logically first stage catalyst bed (i.e., the first bed) of the catalyst bed sequence connected in series.

In the present application, if a plurality of olefins or a plurality of alkylbenzenes are used, the total mole amount of the plurality of olefins or the total mole amount of the plurality of alkylbenzenes is used in calculating the molar ratio.

In the present invention, the range of the inlet temperature of the feed to the switched-in fresh catalyst bed or regenerated catalyst bed can be obtained by formula (1), (1a), (1b), (1c) or formula (2). The inlet temperature of the feed to the fresh catalyst bed or the regenerated catalyst bed can be arbitrarily selected within the range of the inlet temperature obtained by formula (1), (1a), (1b), (1c) or formula (2). For example, if the range of $T_N^{inlet}$ calculated by formula (1) is 40 °C to 119°C, then a temperature can be arbitrarily selected within the range of 40 °C to 119°C as the inlet temperature of the feed to the switched-in fresh catalyst bed or regenerated catalyst bed; for example, 40 °C or 119°C, or any temperature therebetween, can be selected. For another example, if the range of $T_N^{inlet}$ calculated by formula (2) is 107°C to 117°C, then a temperature can be arbitrarily selected within the range of 107 °C to 117°C as the inlet temperature of the feed to the switched-in fresh catalyst bed or regenerated catalyst bed; for example, 107 °C or 117 °C, or any temperature therebetween, can be selected.

In some embodiments, a temperature that is 0.5 to 10 °C lower, preferably 1 to 10 °C lower, and more preferably 1 to 9°C lower, such as a temperature that is 1, 2, 3, 4, 5, 6, 7, 8, 9, or 10 °C lower, than the upper limit of the range of $T_N^{inlet}$ calculated by formula (1), (1a), (1b), (1c) or formula (2) can be selected as the inlet temperature of the feed to the switched-in fresh catalyst bed or regenerated catalyst bed. For example, if the range of $T_N^{inlet}$ calculated by formula (1) is 40 °C to 119°C, then a temperature in the range of 109°C to 118.5 °C can be selected as the inlet temperature of the feed to the switched-in fresh catalyst bed or regenerated catalyst bed.

In some embodiments, if the outlet temperature of the preceding bed immediately upstreaming the switched-in fresh catalyst bed or regenerated catalyst bed is higher than the upper limit of the range of $T_N^{inlet}$ calculated by formula (1), (1a), (1b), (1c) or formula (2), the inlet temperature of the feed to the switched-in fresh catalyst bed or regenerated catalyst bed can be adjusted to be within the range of $T_N^{inlet}$ calculated by formula (1), (1a), (1b), (1c) or formula (2) by cooling.

In some embodiments, if the outlet temperature of the preceding bed immediately upstreaming the switched-in fresh catalyst bed or the regenerated catalyst bed is within the range of $T_N^{inlet}$ calculated by formula (1), (1a), (1b), (1c) or formula (2), the outlet temperature of the preceding bed can be used as the inlet temperature of the feed to the switched-in fresh catalyst bed or regenerated catalyst bed.

In some embodiments, if the outlet temperature of the preceding bed immediately upstream of the switched-in fresh catalyst bed or regenerated catalyst bed is lower than the lower limit of the range of $T_N^{inlet}$ calculated by formula (1), (1a), (1b), (1c) or formula (2), the inlet temperature of the feed to the switched-in fresh catalyst bed or regenerated catalyst bed can be adjusted to be within the range of $T_N^{inlet}$ calculated by formula (1), (1a), (1b), (1c) or formula (2) by heating.

In the present invention, the inlet temperature of the newly switched-in fresh catalyst bed or regenerated catalyst bed can be finely controlled by formula (1), (1a), (1b), (1c) or formula (2), so that not only the discharge temperature of the bed can be controlled to be lower than or equal to 130 °C, but also unnecessary energy consumption can be avoided. Specifically, there is no need to significantly reduce the inlet temperature of the fresh catalyst bed, otherwise there may be serious energy waste. For example, when it is calculated that the inlet temperature of the newly switched-in reactor $R_i$ needs to be controlled at 40-100 °C, since the outlet temperature of the reactor (catalyst bed) preceding $R_i$ is about 120-130 °C, the closer the inlet temperature of $R_i$ is set to the calculated upper limit 100 °C, the smaller the required cooling amount is, and the more energy-saving it is.

In the method of the present invention, in some embodiments, the newly switched-in fresh catalyst bed or regenerated catalyst bed can be located at any stage in the catalyst bed sequence connected in series in the epoxidation reaction zone, for example, it can be located at the logically first stage, the logically last stage or any stage therebetween. However, preferably, in some embodiments of the present invention, the newly switched-in fresh catalyst bed or regenerated catalyst bed is located at the logically N-th stage (i.e., being the N-th bed) in the catalyst bed sequence connected in series, wherein the sum of the temperature rises of the N-1 catalyst beds upstreaming the newly switched-in fresh catalyst bed or regenerated catalyst bed satisfies the following formula (3):

$$\sum_{i=1}^{N-1}(T_i^{outlet}-T_i^{inlet}) > \frac{1370}{a_1+1.38b_1}-90 \qquad (3)$$

preferably, satisfies the following formula (4):

$$\sum_{i=1}^{N-1}(T_i^{outlet}-T_i^{inlet}) > \frac{1370}{a_1+1.38b_1}-50 \qquad (4);$$

wherein, in formula (3) and formula (4), N, $T_i^{outlet}$, $T_i^{inlet}$, $\sum_{i=1}^{N-1}(T_i^{outlet}-T_i^{inlet})$, $a_1$ and $b_1$ are as defined in formula (1).

In some embodiments, the newly switched-in fresh catalyst bed or regenerated catalyst bed can be located at the logically last stage of the catalyst beds connected in series in the epoxidation reaction zone.

In some embodiments, the alkylene oxide can be selected from the group consisting of propylene oxide, ethylene oxide and butylene oxide, preferably propylene oxide.

In some embodiments, the olefin can be selected from the group consisting of ethylene, propylene, and butene, more preferably propylene.

In some embodiments, the alkylbenzene hydroperoxide is cumene hydroperoxide.

In some embodiments, the alkylbenzene can be at least one selected from ethylbenzene, cumene, butylbenzene or a combination thereof, preferably cumene.

In a preferred embodiment, the olefin is propylene, and the alkylbenzene hydroperoxide is cumene hydroperoxide. In a preferred embodiment, the olefin is propylene, the alkylbenzene hydroperoxide is cumene hydroperoxide, and the alkylbenzene is cumene.

In the method of the present invention, the olefin, such as propylene, can be fed as a propylene stream.

In some embodiments, the alkylbenzene hydroperoxide is fed in the form of a solution, wherein the solution comprises alkylbenzene hydroperoxide and alkylbenzene, wherein the alkylbenzene is a solvent or a dispersant. In some embodiments, the alkylbenzene can be at least one selected from ethylbenzene, cumene, butylbenzene and a combination thereof, preferably cumene.

The method of the present invention has no particular requirements on the concentration of the solution comprising alkylbenzene hydroperoxide and alkylbenzene. In some embodiments, the concentration of alkylbenzene in the solution can be 5wt% to 95wt%, preferably 40wt% to 90wt%, for example, can be 40wt%, 50wt%, 60wt%, 70wt%, 80wt% or 90wt%.

In the method of the present invention, there is no particular requirements on the molar ratio $a_1$ of the olefin to the alkylbenzene hydroperoxide in the feed, as long as the alkylene oxide can be prepared. In some embodiments, the molar ratio of the olefin to the alkylbenzene hydroperoxide can be 2 to 50, preferably 2 to 12; for example, the molar ratio of the olefin to the alkylbenzene hydroperoxide can be 2, 4, 6, 8, 10, 12, 16, 18, 20, 22, 24, 26, 28, 30, 32, 34, 36, 38, 40, 42, 44, 46, 48, 50, or a range formed by any two of the above values.

In the method of the present invention, there is no particular requirements on the molar ratio $b_1$ of alkylbenzene to alkylbenzene hydroperoxide in the feed, as long as the alkylene oxide can be prepared. In some embodiments, the molar ratio $b_1$ of alkylbenzene to alkylbenzene hydroperoxide in the feed can be 1 to 30, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or a range formed by any two of the above values.

In some embodiments, the propylene can be preheated before being fed. Various heating means commonly known in the art can be used for preheating. For example, the preheating means include but are not limited to electric heating, steam heating, hot water heating, heat exchange, etc. In some embodiments, the propylene can be preheated to 0-130 °C, such as 0 °C, 10 °C, 20 °C, 30 °C, 40 °C, 50 °C, 60 °C, 70 °C, 80 °C, 90 °C, 100 °C, 110 °C, 120 °C, 130 °C, or a range formed by any two of the above values.

In the method of the present invention, in some embodiments, the propylene and the solution comprising alkylbenzene hydroperoxide and alkylbenzene can be fed into the logically first stage catalyst bed separately, or the propylene and the solution comprising alkylbenzene hydroperoxide and alkylbenzene can be firstly mixed to obtain a mixture, and then fed into the logically first stage catalyst bed. In some embodiments, preferably, the propylene and the solution comprising alkylbenzene hydroperoxide and alkylbenzene are firstly mixed and then fed into the catalyst bed.

In the method of the present invention, the discharge from each catalyst bed is sent to the inlet of the next catalyst bed. In the method of the present invention, if required, the discharge from each catalyst bed can be cooled to achieve the desired temperature (i.e., the inlet temperature of the next catalyst bed). In the method of the present invention, in some embodiments, propylene can be preheated to the inlet temperature of the logically first stage catalyst bed, and the discharge from each catalyst bed is cooled to the inlet temperature of the next bed.

For the cooling of the discharge of a catalyst bed, the present invention can adopt any cooling means generally known in the art. In some embodiments, the cooling can include but is not limited to air cooling, water cooling or heat integration.

Methods for preparing alkylene oxide by reacting an olefin and an alkylbenzene hydroperoxide in the presence of a catalyst in a solution are known in the art. Those skilled in the art can suitably select the inlet temperature of each catalyst bed, provided that the outlet temperature of each catalyst bed does not exceed 130 °C. In some embodiments, the inlet temperature of each catalyst bed can be in the range of 0 to 130 °C. In the method of the present invention, those skilled in the art can suitably select the pressure of each catalyst bed, for example, the pressure of each catalyst bed can be 2 to 20 MPag.

In some embodiments, the inlet temperature of each catalyst bed can be in the range of 40 to 130 °C and the outlet temperature of each catalyst bed does not exceed 130 °C, and the pressure of each catalyst bed can be in the range of 3 to 12 MPag. For example, the inlet temperature of each catalyst bed can be 40 °C, 50 °C, 60 °C, 70 °C, 80 °C, 90 °C, 100 °C, 110 °C, 120 °C or 130 °C, or a range formed by any two of the above values; and/or the pressure of each catalyst bed can be 3 MPag, 4 MPag, 5 MPag, 6 MPag, 7 MPag, 8 MPag, 9 MPag, 10 MPag, 11 MPag or 12 MPag, or a range formed by any two of the above values.

In the method of the present invention, the temperature of the epoxidation reaction can be 0-200 °C, and the pressure can be 2-20 MPag; preferably, the temperature of the epoxidation reaction can be 40-130 °C, and the pressure can be 3-12 MPag.

For the method of the present invention, in some embodiments, the inlet temperature of the catalyst bed and the pressure of the catalyst bed are such that the epoxidation reaction carried out in the catalyst bed is a neat liquid phase reaction.

As known in the art, the epoxidation reaction of the present invention is a highly exothermic reaction. Therefore, if the inlet temperature of the catalyst bed is $T_i^{inlet}$ and the outlet temperature is $T_i^{outlet}$, then $T_i^{outlet} \geq T_i^{inlet}$.

In the present invention, in some embodiments, when the catalyst bed (reactor) does not have a temperature rise, that is, when the inlet temperature $T_i^{inlet}$ is equal to the outlet temperature $T_i^{outlet}$, it can be determined that the catalyst bed is deactivated and needs to be switched out.

In some embodiments, the epoxidation reaction zone can include 2 to 20, preferably 3 to 20, preferably 3 to 10, more preferably 3 to 7, for example 3 to 6, catalyst beds connected in series. In yet some other embodiments, the epoxidation reaction zone can include 2 to 10, preferably 2 to 5 catalyst beds connected in series. For example, the epoxidation reaction zone can include 2, 3, 4, 5, 6, 7, 8, 9, 10, 11, 12, 13, 14, 15, 16, 17, 18, 19 or 20 catalyst beds connected in series. The catalyst beds in the epoxidation reaction zone are each loaded with an epoxidation catalyst. Each catalyst bed in the epoxidation reaction zone can be loaded with same amount or different amounts of epoxidation catalyst.

The epoxidation catalyst can be any catalyst known in the art that can catalyze epoxidation reactions. Preferably, the epoxidation catalyst is a titanium-containing catalyst, more preferably at least one selected from titanium silicon molecular sieve, titanium dioxide and combinations thereof.

In some embodiments of the present invention, the catalyst used in the epoxidation reaction of the present invention can be the catalyst disclosed in CN104437618B.

In some embodiments of the present invention, the catalyst bed of the present invention can include particles of epoxidation catalyst. The catalyst particles can have any particle shape and size commonly known or used in the art.

The catalyst bed of the present invention can have any bed height and any bed shape. In some embodiments of the present invention, the catalyst bed of the present invention can have a bed height and a bed shape commonly known or used in the art.

In some embodiments, the catalyst bed of the present invention can be a fixed bed catalyst bed.

In the present invention, the reactant stream can flow through the catalyst bed from top to bottom, or flow through the catalyst bed from bottom to top. Preferably, the reactant stream flows through the catalyst bed from bottom to top.

For example, the stream can enter the catalyst bed from the top of the catalyst bed, flow through the catalyst bed, and exit from the bottom of the catalyst bed.

In the method of the present invention, the feed comprising an olefin, an alkylbenzene hydroperoxide and an alkylbenzenes is fed in at the catalyst bed located at the logically first stage of the reaction zone.

In the method of the present invention, the reaction product (discharge) of the logically preceding stage catalyst bed enters the logically next stage catalyst bed as the feed of said logically next stage catalyst bed.

In some embodiments, based on the total amount of catalyst present in the epoxidation reaction zone, the mass space velocity of the alkylbenzene hydroperoxide can be 0.1-3 h$^{-1}$, preferably 0.2-2 h$^{-1}$, for example, ca be 0.2 h$^{-1}$, 0.3 h$^{-1}$, 0.4 h$^{-1}$, 0.5 h$^{-1}$, 0.6 h$^{-1}$, 0.7 h$^{-1}$, 0.8 h$^{-1}$, 0.9 h$^{-1}$, 1 h$^{-1}$, 1.1 h$^{-1}$, 1.2 h$^{-1}$, 1.3 h$^{-1}$, 1.4 h$^{-1}$, 1.5 h$^{-1}$, 1.6 h$^{-1}$, 1.7 h$^{-1}$, 1.8 h$^{-1}$, 1.9 h$^{-1}$ or 2 h$^{-1}$.

In the method of the present invention, the fresh catalyst bed refers to a bed comprising fresh catalyst, and the fresh catalyst refers to a fresh catalyst that has not been used. In the method of the present invention, the regenerated catalyst bed refers to a bed in which the performance of the catalyst is regenerated, which is obtained by catalyst regeneration treatment after the catalyst bed is deactivated. Various methods for regenerating catalysts known in the art can be used to regenerate the deactivated catalyst.

In the present invention, the term "catalyst bed" can be used interchangeably with the term "reactor". That is, in the present invention, one catalyst bed is configured as one reactor.

Another aspect of the present invention provides a system for preparing alkylene oxide, the system comprises an epoxidation reaction zone comprising at least two catalyst beds connected in series, and a temperature controller;

wherein a feed comprising an olefin, an alkylbenzene hydroperoxide and an alkylbenzene enters the epoxidation reaction zone to carry out an epoxidation reaction to obtain a product comprising an alkylene oxide, wherein the feed enters the logically first stage catalyst bed of the at least two catalyst beds connected in series and passes sequentially through the at least two catalyst beds connected in series,

wherein, the temperature controller controls the inlet temperature of the feed to the newly switched-in fresh catalyst bed or regenerated catalyst bed such that the inlet temperature satisfies the following formula (1):

$$40°C \leq T_N^{inlet} \leq 130°C + \sum_{i=1}^{N-1}(T_i^{outlet} - T_i^{inlet}) - \frac{1370}{a_1 + 1.38b_1} \qquad (1);$$

in formula (1), $T_N^{inlet}$ represents the inlet temperature of the fresh catalyst bed or the regenerated catalyst bed, N represents that the switched-in fresh catalyst bed or regenerated catalyst bed is located at the logically N-th stage in the catalyst beds connected in series, $T_i^{outlet}$ represents the outlet temperature of the catalyst bed at the logically i-th stage in the catalyst beds connected in series after the fresh catalyst bed or the regenerated catalyst bed is switched in, $T_i^{inlet}$ represents the inlet temperature of the catalyst bed at the logically i-th stage, $\sum_{i=1}^{N-1}(T_i^{outlet} - T_i^{inlet})$ represents the sum of the temperature rises of all catalyst beds upstreaming the switched-in fresh catalyst bed or regenerated catalyst bed, $a_1$ represents the molar ratio of the olefin to the alkylbenzene hydroperoxide in the feed, and $b_1$ represents the molar ratio of the alkylbenzene to the alkylbenzene hydroperoxide in the feed.

In some embodiments, in the system of the present invention, the alkylene oxide, the catalyst beds in the epoxidation reaction zone, the catalysts in the catalyst beds, the olefin, the alkylbenzene hydroperoxide and the alkylbenzene, the epoxidation reaction, the formula (1) (including $T_N^{inlet}$, N, $T_i^{outlet}$, $T_i^{inlet}$, $\sum_{i=1}^{N-1}(T_i^{outlet} - T_i^{inlet})$, $a_1$ and $b_1$) can be as described above with respect to the methods of the present invention.

In some embodiments, an olefin feed pipeline and an alkylbenzene hydroperoxide feed pipeline connected to the logically first stage catalyst bed are arranged before the logically first stage catalyst bed.

In some embodiments, a preheating element is arranged on the olefin feed pipeline; preferably, the preheating element is a heater or a heat exchanger.

In some embodiments, a cooling element is arranged on the connecting pipeline between two adjacent catalyst beds.

In some embodiments, the cooling element can be an air cooling element, a water cooling element, or a heat integration element.

In some embodiments, the epoxidation reaction zone can include 2 to 20, preferably 3 to 20, preferably 3 to 10, preferably 3 to 7, preferably 3 to 6 catalyst beds connected in series. For example, the epoxidation reaction zone can include 2, 3, 4, 5, 6, 7, 8, 9, 10, 11, 12, 13, 14, 15, 16, 17, 18, 19 or 20 catalyst beds connected in series.

In some embodiments, the epoxidation reaction zone can include 2 to 10, preferably 2 to 5 catalyst beds connected in series.

In some embodiments, the newly switched-in fresh catalyst bed or regenerated catalyst bed is located at the logically last stage of the epoxidation reaction zone.

In some embodiments, the inlet and outlet of each catalyst bed are each independently provided with a switching valve, for switching in or switching out of catalyst bed.

For example, the catalyst bed at the logically first stage of the epoxidation reaction zone is switched out after deactivation, and a fresh catalyst bed or a regenerated catalyst bed is switched into the epoxidation reaction zone, for example, switched in the logically last stage of the epoxidation reaction zone.

**In** some embodiments, the system further comprises one or more fresh catalyst bed(s) or regenerated catalyst bed(s) to be switched in.

**In** some embodiments, the catalyst bed at a logical stage in the epoxidation reaction zone can be switched out according to the logical stage after deactivation, and the fresh catalyst bed or the regenerated catalyst bed can be switched into the epoxidation reaction zone after the deactivated catalyst bed is switched out, for example, switched in the epoxidation reaction zone at the logically last stage.

**In** some embodiments, for the system of the present invention, the temperature controller can control the inlet temperature of the feed to the switched-in fresh catalyst bed or regenerated catalyst bed such that the inlet temperature satisfies the following formula (1a):

$$90°C + \sum_{i=1}^{N-1}(T_i^{outlet} - T_i^{inlet}) - \frac{1370}{a_1 + 1.38b_1} \leq T_N^{inlet} \leq 130°C + \sum_{i=1}^{N-1}(T_i^{outlet} - T_i^{inlet}) - \frac{1370}{a_1 + 1.38b_1} \qquad (1a)$$

preferably, satisfies the following formula (1b):

$$100°C + \sum_{i=1}^{N-1}(T_i^{outlet} - T_i^{inlet}) - \frac{1370}{a_1 + 1.38b_1} \leq T_N^{inlet} \leq 130°C + \sum_{i=1}^{N-1}(T_i^{outlet} - T_i^{inlet}) - \frac{1370}{a_1 + 1.38b_1} \qquad (1b)$$

preferably, satisfies the following formula (1c):

$$110°C + \sum_{i=1}^{N-1}(T_i^{outlet} - T_i^{inlet}) - \frac{1370}{a_1 + 1.38b_1} \leq T_N^{inlet} \leq 130°C + \sum_{i=1}^{N-1}(T_i^{outlet} - T_i^{inlet}) - \frac{1370}{a_1 + 1.38b_1} \qquad (1c)$$

more preferably, the temperature controller can control the inlet temperature of the feed to the switched-in fresh catalyst bed or regenerated catalyst bed such that the inlet temperature satisfies the following formula (2):

$$120\text{°C}+\sum_{i=1}^{N-1}(T_i^{outlet}-T_i^{inlet})-\frac{1370}{a_1+1.38b_1}\leq T_N^{inlet}\leq 130\text{°C}+\sum_{i=1}^{N-1}(T_i^{outlet}-T_i^{inlet})-\frac{1370}{a_1+1.38b_1}\qquad(2).$$

In some embodiments, for the system of the present invention, the newly switched-in fresh catalyst bed or regenerated catalyst bed is located at the logically N-th stage in the catalyst beds connected in series, wherein the sum of the temperature rises of the N-1 catalyst beds upstreaming it satisfies the following formula (3):

$$\sum_{i=1}^{N-1}(T_i^{outlet}-T_i^{inlet})>\frac{1370}{a_1+1.38b_1}-90\qquad(3);$$

preferably, satisfies the following formula (4):

$$\sum_{i=1}^{N-1}(T_i^{outlet}-T_i^{inlet})>\frac{1370}{a_1+1.38b_1}-50\qquad(4).$$

In the system of the present invention, in formula (1a), formula (1b), formula (1c), formula (2), formula (3) and formula (4), $T_N^{inlet}$, N, $T_i^{outlet}$, $T_i^{inlet}$, $\sum_{i=1}^{N-1}(T_i^{outlet}-T_i^{inlet})$, , $a_1$ and $b_1$ are as defined in formula (1).

In some embodiments, in the system of the present invention, the inlet and outlet of each catalyst bed are each independently provided with a temperature detector. The temperature detector can detect the inlet temperature and outlet temperature of each catalyst bed, and can send the detected temperatures to the temperature controller. For the temperature detector, various detectors generally known in the art can be used, as long as the required temperature detection function can be achieved. For example, the temperature detector can be a thermocouple capable of transmitting a signal.

In the system of the present invention, the temperature controller can control the inlet temperature of the feed to the switched-in fresh catalyst bed or regenerated catalyst bed through a cooling element; preferably, the temperature controller controls the inlet temperature of the feed to the switched-in fresh catalyst bed or regenerated catalyst bed through a cooling element arranged on a connecting pipeline between two adjacent catalyst beds; more preferably, the cooling element is electrically connected to the temperature controller.

For example, in some embodiments, the temperature controller can collect the temperatures detected by the temperature detectors and determine the range of $T_N^{inlet}$ according to the inputted values of N, $a_1$ and $b_1$ according to formula (1), (1a), (1b), (1c) or formula (2), so that the temperature controller can reduce the temperature $T_N^{inlet}$ of the feed to be introduced into the newly switched-in fresh catalyst bed or regenerated catalyst bed through a cooling element so as to meet the requirement of formula (1), (1a), (1b), (1c) or formula (2).

In some embodiments, the cooling element can be an air cooling element, a water cooling element or a heat integration element. The temperature controller of the present invention can control the fan speed of the air cooling element, can control the valve of the water cooling element to control the water flow rate, or can control the valve of the heat integration element to control the bypass flow rate.

The catalyst beds in the epoxidation reaction zone can each be loaded with an epoxidation catalyst. The epoxidation catalyst can be any catalyst known in the art that can perform the epoxidation reaction. Preferably, the epoxidation catalyst is a titanium-containing catalyst, more preferably at least one selected from titanium silicon molecular sieve, titanium dioxide and a combination thereof.

In some embodiments, all temperature detectors provided at the inlets and outlets of the catalyst beds are connected to the temperature controller, for example, electrically connected.

In some embodiments, the cooling element(s) are connected to the temperature controller, such as electrically connected.

In some embodiments, the temperature controller reduces the inlet temperature of the switched-in fresh catalyst bed or regenerated catalyst bed located at logically N-th stage through the arranged cooling element, so as to control the inlet temperature within a desired range.

For the temperature controller in the present application, various controllers generally known in the art can be used, as long as the control functions disclosed in the present application can be achieved.

In some embodiments, preferably, the system of the present invention is used to carry out the method of the present invention.

The endpoints of the ranges and any values disclosed in the present invention are not limited to the precise ranges or values, and these ranges or values should be understood to include values close to these ranges or values. For numerical ranges, combinations with each other can be made between the endpoint values of each range, between the endpoint values of each range and the individual point values, and between the individual point values to obtain one or more new numerical ranges, which should be regarded as specifically disclosed herein. Herein, in principle, each technical solution can be combined with each other to obtain new technical solutions, which should also be regarded as specifically disclosed herein.

Compared with the prior art, the present invention can provide the following beneficial effects:

(1) The system or method of the present invention can effectively control the temperature of the catalyst bed loaded with fresh or regenerated catalyst (alkylene oxide reaction temperature) newly switched-in into the system, thereby avoiding reaction runaway caused by excessive increase in reaction temperature;
(2) The system or method of the present invention can reasonably control the temperature rise of the reaction system, thereby effectively avoiding the occurrence of side reactions in the epoxidation reaction and improving the selectivity to the target product propylene oxide;
(3) The system or method of the present invention can reasonably control the inlet temperature of a newly switched-in catalyst bed so as to reduce the required cooling amount, thereby saving energy.

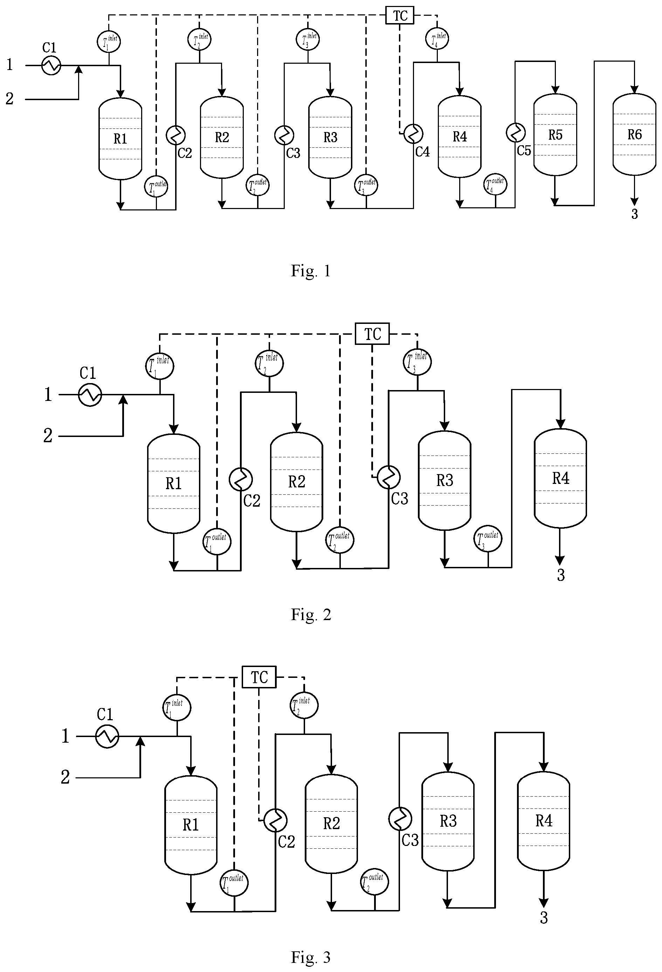

Fig. 1 to Fig. 5 are schematic diagrams of some embodiments of the system of the present invention, wherein the epoxidation reaction zone includes different numbers of reactors (i.e., includes different total numbers of epoxidation catalyst beds).

In Figs. 1 to 3, 1 is the feedstock propylene; 2 is the cumene hydroperoxide solution comprising alkylbenzene; 3 is the epoxidation reaction product; C1 is the propylene feed preheating element; C2 to C5 respectively represent the cooling elements between adjacent catalyst beds; R1 to R6 respectively represent catalyst bed 1, catalyst bed 2, catalyst bed 3, catalyst bed 4, catalyst bed 5, and catalyst bed 6. $T_i^{inlet}$ (i=1/2/3/4) represents the inlet temperature detected by the temperature detector at the inlet of the catalyst bed; $T_i^{outlet}$ (i=1/2/3/4) represents the outlet temperature detected by the temperature detector at the outlet of the catalyst bed. When the catalyst bed at the logically first stage of the epoxidation reaction zone is deactivated, it is switched out and a fresh catalyst bed or a regenerated catalyst bed ($R_4$ in Fig. 1; $R_3$ in Fig. 2; $R_2$ in Fi. 3) is switched in. TC represents a temperature controller, which adjusts the inlet stream temperature $T_N^{inlet}$ of the catalyst bed by controlling the cooling element at the inlet of the switched-in catalyst bed, wherein $T_N^{inlet}$ satisfies, for example, the following formula:

$$40°C \leq T_N^{inlet} \leq 130°C + \sum_{i=1}^{N-1}(T_i^{outlet} - T_i^{inlet}) - \frac{1370}{a_1 + 1.38b_1} ;$$

so that the outlet stream temperature $T_N^{outlet}$ of the switched-in catalyst bed is lower than or equal to 130 °C.

Taking Fig. 3 as an example, the feedstock propylene 1 is preheated by the propylene feed preheating element C1, and then mixed with the cumene hydroperoxide solution comprising alkylbenzene and passed from the top into the catalyst bed 1 (the logically first stage catalyst bed in the epoxidation reaction zone), which is represented as R1 in the figure. The newly switched-in fresh catalyst bed or regenerated catalyst bed is represented as R2, which is located downstream of R1. The stream discharging from the bottom outlet of the catalyst bed 1 is cooled by the cooling element C2 and passed from the top into the catalyst bed 2. Since the activity of the fresh or regenerated catalyst loaded in the newly switched-in bed is relatively high, the inlet temperature of the newly switched-in catalyst bed is controlled to be within the range defined by formula (1) through the control manner of the present invention, so as to prevent the reactor outlet temperature from being too high and the degree of side reactions from being aggravated. Finally, stream 3 containing propylene oxide is obtained at the outlet of the reaction system.

## BRIEF DESCRIPTION OF THE DRAWINGS

Fig. 1 is a schematic diagram of an embodiment of the system of the present invention, wherein the epoxidation reaction zone comprises 6 reactors, that is, the total number of epoxidation catalyst beds is 6.

Fig. 2 is a schematic diagram of an embodiment of the system of the present invention, wherein the epoxidation reaction zone comprises 4 reactors, that is, the total number of epoxidation catalyst beds is 4.

Fig. 3 is a schematic diagram of an embodiment of the system of the present invention, wherein the epoxidation reaction zone comprises 4 reactors, that is, the total number of catalyst beds is 4.

Fig. 4 is a schematic diagram of an embodiment of the system of the present invention, wherein the reaction zone comprises 6 reactors (N=6), that is, the total number of epoxidation catalyst beds is 6.

Fig. 5 is a schematic diagram of an embodiment of the system of the present invention, wherein the reaction zone comprises 5 reactors (N=5), that is, the total number of epoxidation catalyst beds is 5.

## DETAILED DESCRIPTION

The present invention is described in detail below in conjunction with specific embodiments. It is necessary to point out here that the following embodiments are only used to further illustrate the present invention and cannot be understood as limitations to the scope of protection of the present invention. Some non-essential improvements and adjustments made by those skilled in the art to the present invention based on the contents of the present invention still belong to the scope of protection of the present invention.

It should also be noted that the various specific technical features described in the following specific embodiments can be combined in any suitable manner without contradiction. To avoid unnecessary repetition, the present invention will not further describe various possible combinations.

In addition, the various embodiments of the present invention may be arbitrarily combined as long as they do not violate the concept of the present invention. The technical solutions thus formed are part of the original disclosure of this specification and also fall within the protection scope of the present invention.

Raw materials and reagents used in the Examples and Comparative examples, if not specifically defined, are directly purchased or prepared according to the preparation methods disclosed in the prior art. If necessary, the raw materials and reagents are subjected to conventional pretreatments to meet the requirements of the reaction.

In the following Examples and Comparative examples, the catalyst used was the catalyst prepared according to Example 1 in CN104437618B.

In the Examples of the present invention, liquid chromatography is used to determine the concentration of each component in the outlet steam of the epoxidation reaction zone, and the conversion rate of cumene hydroperoxide and the selectivity of propylene oxide are calculated based on the concentrations; wherein a Shimadzu LC-20 liquid chromatograph with an automatic sampler and a diode array detector (DAD) is used.

## [Example 1]

Propylene oxide was prepared by using the reaction system shown in Fig. 1. Specifically, the following steps were included: the mass flow rate of cumene hydroperoxide stream was 100 kg/h, wherein the mass concentration of cumene hydroperoxide material was 55 wt%, and the mass concentration of cumene was 45 wt%. The mass flow rate of propylene stream was 130.6 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. Epoxidation reactors R1, R2, R3, and R4 were respectively loaded with 11.5 kg of catalyst, and epoxidation reactors R5 and R6 were loaded with 14.7 kg of catalyst; the total mass space velocity of cumene hydroperoxide was 0.73 h⁻¹. The inlet temperature of the epoxidation reactor R4 which was newly switched in the epoxidation reaction system was calculated according to the formula

$$40\text{℃} \le T_N^{inlet} \le 130\text{℃} + \sum_{i=1}^{N-1}(T_i^{outlet} - T_i^{inlet}) - \frac{1370}{a_1 + 1.38b_1}$$ , and the calculated range of $T_N^{inlet}$ was 40 °C~119°C. The R4 inlet temperature was set to be 115°C, and the reactor inlet and outlet temperatures before and after R4 was switched in were shown in Table 1. The outlet stream of the reaction zone was measured and calculated, and the conversion rate of cumene hydroperoxide was 99.2%, and the selectivity of propylene oxide was 98.4%.

Table 1. Reactor inlet and outlet temperatures

| | Before R4 switched-in | | After R4 switched-in | |
|---|---|---|---|---|
| | Inlet temperature,°C | Outlet temperature,°C | Inlet temperature,°C | Outlet temperature,°C |
| R1 | 85 | 126 | 89 | 128 |

(continued)

| | | Before R4 switched-in | | After R4 switched-in | |
|---|---|---|---|---|---|
| | | Inlet temperature,°C | Outlet temperature,°C | Inlet temperature,°C | Outlet temperature,°C |
| | R2 | 78 | 128 | 83 | 128 |
| | R3 | 86 | 126 | 90 | 127 |
| | R4 | - | - | 115 | 128 |
| | R5 | 114 | 124 | 118 | 125 |
| | R6 | 120 | 121 | 123 | 124 |

**[Example 2]**

Propylene oxide was prepared by using the reaction system shown in Fig. 1. Specifically, the following steps were included: the mass flow rate of cumene hydroperoxide stream was 100 kg/h, wherein the mass concentration of cumene hydroperoxide material was 55 wt%, and the mass concentration of cumene was 45 wt%. The mass flow rate of propylene stream was 100.9 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. Epoxidation reactors R1, R2, R3, and R4 were respectively loaded with 5.1 kg of catalyst, and epoxidation reactors R5 and R6 were respectively loaded with 10.2 kg of catalyst; the total mass space velocity of cumene hydroperoxide was 1.35 h$^{-1}$. The inlet temperature of the epoxidation reactor R4 which was newly switched in the epoxidation reaction system was calculated according to the formula

$$40℃ \leq T_N^{inlet} \leq 130℃ + \sum_{i=1}^{N-1}(T_i^{outlet} - T_i^{inlet}) - \frac{1370}{a_1 + 1.38b_1}$$ , and the calculated range of $T_N^{inlet}$ was 40 °C~85°C. The R4 inlet temperature was set to be 83°C, and the reactor inlet and outlet temperatures before and after R4 was switched in were shown in Table 2. The outlet stream of the reaction zone was measured and calculated, and the conversion rate of cumene hydroperoxide was 98.3%, and the selectivity of propylene oxide was 97.2%.

Table 2. Reactor inlet and outlet temperatures

| | | Before R4 switched-in | | After R4 switched-in | |
|---|---|---|---|---|---|
| | | Inlet temperature, °C | Outlet temperature, °C | Inlet temperature, °C | Outlet temperature, °C |
| | R1 | 83 | 123 | 88 | 127 |
| | R2 | 80 | 127 | 81 | 127 |
| | R3 | 81 | 125 | 83 | 126 |
| | R4 | - | - | 83 | 123 |
| | R5 | 93 | 124 | 119 | 125 |
| | R6 | 114 | 127 | 122 | 123 |

**[Example 3]**

Propylene oxide was prepared by using the reaction system shown in Fig. 1. Specifically, the following steps were included: the mass flow rate of cumene hydroperoxide stream was 100 kg/h, wherein the mass concentration of cumene hydroperoxide material was 40 wt%, and the mass concentration of cumene was 60 wt%. The mass flow rate of propylene stream was 52.1 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. Epoxidation reactors R1, R2, R3, and R4 were respectively loaded with 8.3 kg of catalyst, and epoxidation reactors R5 and R6 were respectively loaded with 10.7 kg of catalyst; the total mass space velocity of cumene hydroperoxide was 0.73 h$^{-1}$. The inlet temperature of the epoxidation reactor R4 which was newly switched in the epoxidation reaction system was calculated according to the formula

$$40°C \leq T_N^{inlet} \leq 130°C + \sum_{i=1}^{N-1}(T_i^{outlet} - T_i^{inlet}) - \frac{1370}{a_1 + 1.38b_1}$$ , and the calculated range of $T_N^{inlet}$ was 40 °C~82 °C. The R4 inlet temperature was set to be 81 °C, and the reactor inlet and outlet temperatures before and after R4 was switched in were shown in Table 3. The outlet stream of the reaction zone was measured and calculated, and the conversion rate of cumene hydroperoxide was 98.8%, and the selectivity of propylene oxide was 96.1%.

Table 3. Reactor inlet and outlet temperatures

|  | Before R4 switched-in | | After R4 switched-in | |
| --- | --- | --- | --- | --- |
|  | Inlet temperature, °C | Outlet temperature, °C | Inlet temperature, °C | Outlet temperature, °C |
| R1 | 83 | 128 | 87 | 130 |
| R2 | 81 | 129 | 83 | 128 |
| R3 | 79 | 130 | 83 | 130 |
| R4 | - | - | 81 | 129 |
| R5 | 93 | 128 | 118 | 127 |
| R6 | 113 | 126 | 126 | 126 |

**[Example 4]**

Propylene oxide was prepared by using the reaction system shown in Fig. 2. Specifically, the following steps were included: the mass flow rate of cumene hydroperoxide stream was 100 kg/h, wherein the mass concentration of cumene hydroperoxide material was 40 wt%, and the mass concentration of cumene was 60 wt%. The mass flow rate of propylene stream was 95 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. Epoxidation reactors R1, R2 and R3 were respectively loaded with 8.3 kg of catalyst, and epoxidation reactor R4 was loaded with 10.7 kg of catalyst; the total mass space velocity of cumene hydroperoxide was 1.12 h$^{-1}$. The inlet temperature of the epoxidation reactor R3 which was newly switched in the epoxidation reaction system was calculated according to the formula $$40°C \leq T_N^{inlet} \leq 130°C + \sum_{i=1}^{N-1}(T_i^{outlet} - T_i^{inlet}) - \frac{1370}{a_1 + 1.38b_1}$$ , and the calculated range of $T_N^{inlet}$ was 40 °C~92 °C. The R3 inlet temperature was set to be 90 °C, and the reactor inlet and outlet temperatures before and after R3 was switched in were shown in Table 4. The outlet stream of the reaction zone was measured and calculated, and the conversion rate of cumene hydroperoxide was 98.7%, and the selectivity of propylene oxide was 96.7%.

Table 4. Reactor inlet and outlet temperatures

|  | Before R3 switched-in | | After R3 switched-in | |
| --- | --- | --- | --- | --- |
|  | Inlet temperature, °C | Outlet temperature, °C | Inlet temperature, °C | Outlet temperature, °C |
| R1 | 83 | 127 | 85 | 128 |
| R2 | 81 | 126 | 87 | 127 |
| R3 | - | - | 90 | 128 |
| R4 | 88 | 126 | 119 | 125 |

**[Example 5]**

Propylene oxide was prepared by using the reaction system shown in Fig. 3. Specifically, the following steps were included: the mass flow rate of cumene hydroperoxide stream was 100 kg/h, wherein the mass concentration of cumene hydroperoxide material was 40 wt%, and the mass concentration of cumene was 60 wt%. The mass flow rate of propylene stream was 95 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. Epoxidation reactors R1 and R2 were respectively loaded with 11.7 kg of catalyst, and epoxidation reactors R3 and R4 were respectively loaded with 9.0 kg of catalyst; the total mass space velocity of cumene hydroperoxide was 0.97 h$^{-1}$. The inlet temperature of the epoxidation reactor R2 which was newly switched in the epoxidation reaction system was calculated according to the formula

$$40\text{℃} \le T_N^{inlet} \le 130\text{℃} + \sum_{i=1}^{N-1}(T_i^{outlet} - T_i^{inlet}) - \frac{1370}{a_1 + 1.38b_1},$$

and the calculated range of $T_N^{inlet}$ was 40 °C~70 °C. The R2 inlet temperature was set to be 68 °C, and the reactor inlet and outlet temperatures before and after R2 was switched in were shown in Table 5. The outlet stream of the reaction zone was measured and calculated, and the conversion rate of cumene hydroperoxide was 98.9%, and the selectivity of propylene oxide was 96.3%.

Table 5. Reactor inlet and outlet temperatures

| | Before R2 switched-in | | After R2 switched-in | |
|---|---|---|---|---|
| | Inlet temperature, °C | Outlet temperature, °C | Inlet temperature, °C | Outlet temperature, °C |
| R1 | 63 | 130 | 65 | 129 |
| R2 | - | - | 68 | 130 |
| R3 | 80 | 128 | 108 | 126 |
| R4 | 114 | 126 | 120 | 124 |

**[Example 6]**

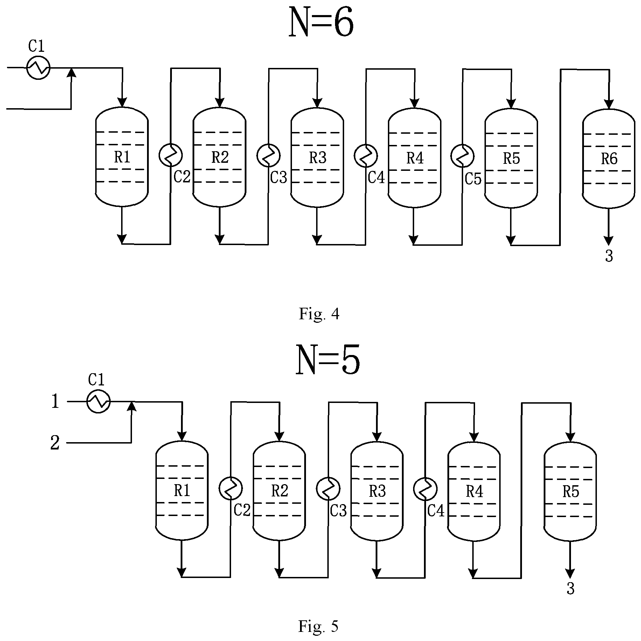

As shown in Fig. 4, the total number of epoxidation reactors was 6. Specifically, the following steps were included: the mass flow rate of cumene hydroperoxide stream was 100 kg/h, wherein the mass concentration of cumene hydroperoxide material was 46 wt%, and the mass concentration of cumene was 54 wt%. The mass flow rate of propylene stream was 109 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. The epoxidation reactors were respectively loaded with 9.6 kg of catalyst; the total mass space velocity of cumene hydroperoxide was 0.80 h$^{-1}$. The stage N of the newly switched in epoxidation reactor should satisfy the formula $\sum_{i=1}^{N-1}(T_i^{outlet} - T_i^{inlet}) > \frac{1370}{a_1 + 1.38b_1} - 50$, and after calculation, N≥4 at this case. The reactor which was newly switched in the epoxidation reaction system was placed at the logically fifth stage. According to calculation based on the formula $120\text{℃} + \sum_{i=1}^{N-1}(T_i^{outlet} - T_i^{inlet}) - \frac{1370}{a_1 + 1.38b_1} \le T_N^{inlet} \le 130\text{℃} + \sum_{i=1}^{N-1}(T_i^{outlet} - T_i^{inlet}) - \frac{1370}{a_1 + 1.38b_1}$ the calculated range of $T_N^{inlet}$ was 107°C~117°C. The inlet temperature of R5 was set to be 110 °C and R5 was switched in the epoxidation reaction system. The reactor inlet and outlet temperatures before and after the R5 was switched in were shown in Table 6. The outlet stream of the reaction zone was measured and calculated, and the conversion rate of cumene hydroperoxide was 99.0%, and the selectivity of propylene oxide was 98.7%.

Table 6. Reactor inlet and outlet temperatures

| | Before R5 switched-in | | After R5 switched-in | |
|---|---|---|---|---|
| | Inlet temperature, °C | Outlet temperature, °C | Inlet temperature, °C | Outlet temperature, °C |
| R1 | 87 | 123 | 91 | 125 |
| R2 | 90 | 125 | 87 | 122 |

(continued)

| | Before R5 switched-in | | After R5 switched-in | |
|---|---|---|---|---|
| | Inlet temperature, °C | Outlet temperature, °C | Inlet temperature, °C | Outlet temperature, °C |
| R3 | 89 | 122 | 90 | 123 |
| R4 | 103 | 120 | 105 | 121 |
| R5 | - | - | 110 | 123 |
| R6 | 108 | 121 | 121 | 122 |

**[Example 7]**

As shown in Fig. 4, the total number of epoxidation reactors was 6. Specifically, the following steps were included: the mass flow rate of cumene hydroperoxide stream was 100 kg/h, wherein the mass concentration of cumene hydroperoxide material was 46 wt%, and the mass concentration of cumene was 54 wt%. The mass flow rate of propylene stream was 109 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. The epoxidation reactors were respectively loaded with 9.6 kg of catalyst; the total mass space velocity of cumene hydroperoxide was 0.80 h$^{-1}$. The stage N of the newly switched in epoxidation reactor should satisfy the formula $\sum_{i=1}^{N-1}(T_i^{outlet} - T_i^{inlet}) > \dfrac{1370}{a_1 + 1.38b_1} - 50$, and after calculation, N≥4 at this case. The reactor which was newly switched in the epoxidation reaction system was placed at the logically fourth stage. According to calculation based on the

formula $120℃ + \sum_{i=1}^{N-1}(T_i^{outlet} - T_i^{inlet}) - \dfrac{1370}{a_1 + 1.38b_1} \leq T_N^{inlet} \leq 130℃ + \sum_{i=1}^{N-1}(T_i^{outlet} - T_i^{inlet}) - \dfrac{1370}{a_1 + 1.38b_1}$, the

calculated range of $T_N^{inlet}$ was 90 °C~100 °C. The inlet temperature of R4 was set to be 95 °C and R4 was switched in the epoxidation reaction system. The reactor inlet and outlet temperatures before and after the R4 was switched in were shown in Table 7. The outlet stream of the reaction zone was measured and calculated, and the conversion rate of cumene hydroperoxide was 99.1%, and the selectivity of propylene oxide was 98.9%.

Table 7. Reactor inlet and outlet temperatures

| | Before R4 switched-in | | After R4 switched-in | |
|---|---|---|---|---|
| | Inlet temperature, °C | Outlet temperature, °C | Inlet temperature, °C | Outlet temperature, °C |
| R1 | 87 | 123 | 91 | 125 |
| R2 | 90 | 125 | 87 | 122 |
| R3 | 89 | 122 | 90 | 123 |
| R4 | - | - | 95 | 124 |
| R5 | 101 | 119 | 112 | 119 |
| R6 | 108 | 121 | 117 | 118 |

**[Example 8]**

As shown in Fig. 5, the total number of epoxidation reactors was 5. The mass flow rate of cumene hydroperoxide stream was 100 kg/h, wherein the mass concentration of cumene hydroperoxide material was 37 wt%, and the mass concentration of cumene was 63 wt%. The mass flow rate of propylene stream was 82 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures so as to keep the outlet temperature of each reactor not exceeding 130 °C. The epoxidation reactors were respectively loaded with 9.3 kg of catalyst; the total mass space velocity of cumene hydroperoxide was 0.80 h$^{-1}$. The stage N of the newly switched in epoxidation reactor should satisfy the formula

$$\sum_{i=1}^{N-1}(T_i^{outlet}-T_i^{inlet}) > \frac{1370}{a_1+1.38b_1}-50$$ , and after calculation, N≥4 at this case. The reactor which was newly switched-in the epoxidation reaction system was placed at the logically fourth stage. According to calculation based on the

formula $$120℃+\sum_{i=1}^{N-1}(T_i^{outlet}-T_i^{inlet})-\frac{1370}{a_1+1.38b_1} \leq T_N^{inlet} \leq 130℃+\sum_{i=1}^{N-1}(T_i^{outlet}-T_i^{inlet})-\frac{1370}{a_1+1.38b_1}$$ , the cal-

culated range of $T_N^{inlet}$ was 100 °C~110 °C. The inlet temperature of R4 was set to be 105 °C and R4 was switched in the epoxidation reaction system. The reactor inlet and outlet temperatures before and after the R4 was switched in were shown in Table 8. The outlet stream of the reaction zone was measured and calculated, and the conversion rate of cumene hydroperoxide was 98.9%, and the selectivity of propylene oxide was 98.3%.

Table 8. Reactor inlet and outlet temperatures

|  | Before R4 switched-in | | After R4 switched-in | |
|---|---|---|---|---|
|  | Inlet temperature, °C | Outlet temperature, °C | Inlet temperature, °C | Outlet temperature, °C |
| R1 | 84 | 121 | 86 | 123 |
| R2 | 87 | 124 | 87 | 123 |
| R3 | 89 | 125 | 91 | 126 |
| R4 | - | - | 105 | 124 |
| R5 | 101 | 121 | 117 | 121 |

**[Example 9]**

Process of Example 6 was repeated, except that the reactor which was newly switched in epoxidation reaction system was placed at the logically sixth stage, i.e., the logically last stage. According to calculation based on the formula

$$120℃+\sum_{i=1}^{N-1}(T_i^{outlet}-T_i^{inlet})-\frac{1370}{a_1+1.38b_1} \leq T_N^{inlet} \leq 130℃+\sum_{i=1}^{N-1}(T_i^{outlet}-T_i^{inlet})-\frac{1370}{a_1+1.38b_1}$$ the calculated

range of $T_N^{inlet}$ was 120 °C~130 °C. Considering that the outlet temperature of R5 was 121 °C, the inlet temperature of R6 can be controlled at 120 ~ 121 °C. The inlet temperature of R6 was set to be 121°C, and R6 was switched in the epoxidation reaction system. The reactor inlet and outlet temperatures before and after the R6 was switched in were shown in Table 9. The outlet stream of the reaction zone was measured and calculated, and the conversion rate of cumene hydroperoxide was 99.1%, and the selectivity of propylene oxide was 98.5%.

Table 9. Reactor inlet and outlet temperatures

|  | Before R6 switched-in | | After R6 switched-in | |
|---|---|---|---|---|
|  | Inlet temperature, °C | Outlet temperature, °C | Inlet temperature, °C | Outlet temperature, °C |
| R1 | 87 | 123 | 91 | 125 |
| R2 | 90 | 125 | 87 | 122 |
| R3 | 89 | 122 | 90 | 123 |
| R4 | 103 | 120 | 105 | 121 |
| R5 | 108 | 121 | 109 | 122 |
| R6 | - | - | 121 | 121 |

**[Comparative Example 1]**

The process of Example 1 was repeated, except that only the catalyst bed was switched, but the inlet temperature of the newly switched in fresh catalyst bed was not controlled, and at this case, the R4 inlet temperature was 126°C, and the R4 outlet temperature was 143°C. The outlet stream of the reaction zone was measured and calculated, and the conversion rate of cumene hydroperoxide was 99.4%, and the selectivity of propylene oxide was 87.5%.

**[Comparative Example 2]**

The process of Example 1 was repeated, except that: the inlet temperature of the newly switched in fresh catalyst bed R4 was controlled by the following formula (5), and the calculated range of $T_N^{inlet}$ was 40 °C to 79°C. The inlet temperature of R4 was set to be 78°C, and the inlet and outlet temperatures of the reactors before and after R4 was switched in were shown in Table 10. The R3 outlet stream needed to be cooled from 126°C to 78°C, and the required cooling amount was higher than that of the solution in Example 1. The outlet stream of the reaction zone was measured and calculated, and the conversion rate of cumene hydroperoxide was 94.8%, and the selectivity of propylene oxide was 98.3%.

$$40°C \leq T_N^{inlet} \leq 130°C\text{-}max(T_i^{outlet}\text{ - }T_i^{inlet}) \qquad \text{formula (5)}$$

Table 10. Reactor inlet and outlet temperatures

| | Before R4 switched-in | | After R4 switched-in | |
|---|---|---|---|---|
| | Inlet temperature, °C | Outlet temperature, °C | Inlet temperature, °C | Outlet temperature, °C |
| R1 | 85 | 126 | 89 | 128 |
| R2 | 78 | 128 | 83 | 128 |
| R3 | 86 | 126 | 90 | 127 |
| R4 | - | - | 78 | 97 |
| R5 | 114 | 124 | 97 | 103 |
| R6 | 120 | 121 | 103 | 104 |

**[Comparative Example 3]**

The process of Example 1 was repeated, except that: the numerator of the last fraction in formula (1) was not 1370, but was 1200. The inlet temperature of the epoxidation reactor R4 which was newly switched in the epoxidation reaction system was calculated according to the formula $40°C \leq T_N^{inlet} \leq 130°C + \sum_{i=1}^{N-1}(T_i^{outlet} - T_i^{inlet}) - \dfrac{1200}{a_1 + 1.38b_1}$,

and the calculated range of $T_N^{inlet}$ was 40 °C to 136°C. Therefore, the R3 outlet stream did not need to be cooled, and the R4 inlet temperature was maintained as the R3 outlet temperature of 126°C. The R4 outlet temperature was 143°C. The reaction zone outlet stream was measured and calculated, and the conversion rate of cumene hydroperoxide was 99.4%, and the selectivity of propylene oxide was 87.5%.

**[Comparative Example 4]**

The process of Example 1 was repeated, except that: the numerator of the last fraction in formula (1) was not 1370, but was 1600. The inlet temperature of the epoxidation reactor R4 which was newly switched in the epoxidation reaction system was calculated according to the formula $40°C \leq T_N^{inlet} \leq 130°C + \sum_{i=1}^{N-1}(T_i^{outlet} - T_i^{inlet}) - \dfrac{1700}{a_1 + 1.38b_1}$,

and the calculated range of $T_N^{inlet}$ was 40 °C to 84 °C. The R4 inlet temperature was set to be 83°C and the R4 outlet temperature was 103°C. In this comparative example, the R3 outlet stream needed to be cooled from 126°C to 83°C, and the required cooling amount was higher than that of the solution in Example 1. The reaction zone outlet stream was measured and calculated, and the conversion rate of cumene hydroperoxide was 95.7%, and the selectivity of propylene oxide was 98.5%.

**[Comparative Example 5]**

The process of Example 6 was repeated, except that: the reactor which was newly switched in the epoxidation reaction system was placed at the logically second stage. According to calculation based on the formula

$$120℃+\sum_{i=1}^{N-1}(T_i^{outlet}-T_i^{inlet})-\frac{1370}{a_1+1.38b_1} \leq T_N^{inlet} \leq 130℃+\sum_{i=1}^{N-1}(T_i^{outlet}-T_i^{inlet})-\frac{1370}{a_1+1.38b_1}$$ , the calcu-

lated range of $T_N^{inlet}$ was 22°C to 32°C. However, when the inlet temperature of R2 was set to be 32 °C, it was found that the epoxidation reaction did not occur and R2 had no temperature rise.

**[Example 10]**

The process of Example 6 was repeated, except that: the stage of the newly switched in epoxidation reactor was different from that of Example 6. According to calculation based on the formula

$$\sum_{i=1}^{N-1}(T_i^{outlet}-T_i^{inlet}) > \frac{1370}{a_1+1.38b_1}-90$$ , N≥3 at this case. The reactor which was newly switched in the epoxidation reaction system was placed at the logically third stage. According to calculation based on the formula

$$120℃+\sum_{i=1}^{N-1}(T_i^{outlet}-T_i^{inlet})-\frac{1370}{a_1+1.38b_1} \leq T_N^{inlet} \leq 130℃+\sum_{i=1}^{N-1}(T_i^{outlet}-T_i^{inlet})-\frac{1370}{a_1+1.38b_1}$$ , the calcu-

lated range of $T_N^{inlet}$ was 57 °C to 67 °C. The R3 inlet temperature was set to be 65°C and R3 was switched in the epoxidation reaction system. The reactor inlet and outlet temperatures before and after R3 was switched in were shown in Table 11. The reaction zone outlet stream was measured and calculated, and the conversion rate of cumene hydroperoxide was 99.1%, and the selectivity of propylene oxide was 97.3%.

Table 11. Reactor inlet and outlet temperatures

|  | Before R3 switched-in | | After R3 switched-in | |
|---|---|---|---|---|
|  | Inlet temperature, °C | Outlet temperature, °C | Inlet temperature, °C | Outlet temperature, °C |
| R1 | 87 | 123 | 91 | 125 |
| R2 | 90 | 125 | 87 | 122 |
| R3 | - | - | 65 | 128 |
| R4 | 89 | 122 | 121 | 123 |
| R5 | 103 | 120 | 120 | 120 |
| R6 | 108 | 121 | 121 | 122 |

**[Example 11]**

As shown in Fig. 5, the total number of epoxidation reactors was 5. The mass flow rate of cumene hydroperoxide stream was 100 kg/h, wherein the mass concentration of cumene hydroperoxide material was 41 wt%, and the mass concentration of cumene was 59 wt%. The mass flow rate of propylene stream was 87 kg/h. To ensure that a neat liquid phase reaction was carried out in the reactors, the pressure of the reactors was controlled to be 6.0 MPaG; the inlet temperature of each reactor was controlled at 40 °C~130 °C, wherein all inlet temperatures were adjusted according to the outlet temperatures to keep the outlet temperature of each reactor not exceeding 130 °C. The epoxidation reactors were respectively loaded with 9.3 kg of catalyst; the total mass space velocity of cumene hydroperoxide was 0.80 h$^{-1}$. The stage

N of the newly switched in epoxidation reactor should satisfy the formula $\sum_{i=1}^{N-1}(T_i^{outlet}-T_i^{inlet}) > \frac{1370}{a_1+1.38b_1}-50$ , and

after calculation, N≥3 at this case. The reactor which was newly switched in the epoxidation reaction system was placed at the logically fourth stage. According to calculation based on the formula

$$90^{\circ}\text{C}+\sum_{i=1}^{N-1}(T_{i}^{outlet}-T_{i}^{inlet})-\frac{1370}{a_{1}+1.38b_{1}}\leq T_{N}^{inlet}\leq 130^{\circ}\text{C}+\sum_{i=1}^{N-1}(T_{i}^{outlet}-T_{i}^{inlet})-\frac{1370}{a_{1}+1.38b_{1}}$$ the calculated

range of $T_{N}^{inlet}$ was 80 °C~120 °C. The inlet temperature of R4 was set to be 95 °C and R4 was switched in the epoxidation reaction system. The reactor inlet and outlet temperatures before and after the R4 was switched in were shown in Table 12. The outlet stream of the reaction zone was measured and calculated, and the conversion rate of cumene hydroperoxide was 98.4%, and the selectivity of propylene oxide was 99.1%.

Table 12. Reactor inlet and outlet temperatures

|  | Before R4 switched-in | | After R4 switched-in | |
|---|---|---|---|---|
|  | Inlet temperature, °C | Outlet temperature, °C | Inlet temperature, °C | Outlet temperature, °C |
| R1 | 73 | 112 | 75 | 114 |
| R2 | 65 | 110 | 68 | 113 |
| R3 | 61 | 107 | 65 | 108 |
| R4 | - | - | 95 | 108 |
| R5 | 105 | 115 | 106 | 107 |

**[Example 12]**

As shown in Fig. 4, the total number of epoxidation reactors was 6. The mass flow rate of cumene hydroperoxide stream was 100 kg/h, wherein the mass concentration of cumene hydroperoxide material was 53 wt%, and the mass concentration of cumene was 47 wt%. The mass flow rate of propylene stream was 81 kg/h. To ensure that a neat liquid phase reaction was carried out in the reactors, the pressure of the reactors was controlled to 6.0 MPaG; the inlet temperature of each reactor was controlled at 40 °C~130 °C, wherein all inlet temperatures were adjusted according to the outlet temperatures to keep the outlet temperature of each reactor not exceeding 130 °C. The epoxidation reactors were respectively loaded with 9.6 kg of catalyst; the total mass space velocity of cumene hydroperoxide was 0.80 h$^{-1}$. The stage N of the newly switched in epoxidation reactor should satisfy the formula $\sum_{i=1}^{N-1}(T_{i}^{outlet}-T_{i}^{inlet})>\frac{1370}{a_{1}+1.38b_{1}}-50$, and after calculation, N≥3 at this case. The reactor which was newly switched in the epoxidation reaction system was placed at the logically fourth stage. According to calculation based on the formula

$$100^{\circ}\text{C}+\sum_{i=1}^{N-1}(T_{i}^{outlet}-T_{i}^{inlet})-\frac{1370}{a_{1}+1.38b_{1}}\leq T_{N}^{inlet}\leq 130^{\circ}\text{C}+\sum_{i=1}^{N-1}(T_{i}^{outlet}-T_{i}^{inlet})-\frac{1370}{a_{1}+1.38b_{1}}$$ , the calcu-

lated range of $T_{N}^{inlet}$ was 52 °C~82 °C. The inlet temperature of R4 was set to be 55 °C and R4 was switched in the epoxidation reaction system. The reactor inlet and outlet temperatures before and after the R4 was switched in were shown in Table 13. The outlet stream of the reaction zone was measured and calculated, and the conversion rate of cumene hydroperoxide was 98.7%, and the selectivity of propylene oxide was 99.3%.

Table 13. Reactor inlet and outlet temperatures

|  | Before R4 switched-in | | After R4 switched-in | |
|---|---|---|---|---|
|  | Inlet temperature, °C | Outlet temperature, °C | Inlet temperature, °C | Outlet temperature, °C |
| R1 | 67 | 101 | 71 | 102 |
| R2 | 65 | 105 | 68 | 107 |
| R3 | 55 | 103 | 59 | 105 |
| R4 | - | - | 55 | 100 |
| R5 | 67 | 100 | 95 | 102 |
| R6 | 93 | 108 | 101 | 102 |

The present invention has been described in detail above in conjunction with specific embodiments and exemplary examples, but these descriptions cannot be construed as limitations to the present invention. Those skilled in the art understand that, without departing from the spirit and scope of the present invention, a variety of equivalent substitutions, modifications or improvements can be made to the technical solutions and its embodiments of the present invention, all of which fall within the scope of the present invention. The scope of protection of the present invention shall be determined by the appended claims.

**Claims**

1. A method for preparing alkylene oxide, including feeding a feed comprising an olefin, an alkylbenzene hydroperoxide and an alkylbenzene into an epoxidation reaction zone comprising at least two catalyst beds connected in series to carry out an epoxidation reaction to obtain a reaction product comprising an alkylene oxide, wherein the feed enters the logically first stage catalyst bed of the at least two catalyst beds connected in series and passes sequentially through the at least two catalyst beds connected in series, wherein when a catalyst bed, preferably the logically first stage catalyst bed, is deactivated, it is switched out and a fresh catalyst bed or a regenerated catalyst bed is switched in;

   wherein the inlet temperature of the feed entering the switched-in fresh catalyst bed or regenerated catalyst bed satisfies the following formula (1) such that the temperature of the discharge leaving the switched-in fresh catalyst bed or regenerated catalyst bed is lower than or equal to 130 °C:

   $$40°C \leq T_N^{inlet} \leq 130°C + \sum_{i=1}^{N-1}(T_i^{outlet} - T_i^{inlet}) - \frac{1370}{a_1 + 1.38b_1} \qquad (1)$$

   in formula (1), $T_N^{inlet}$ represents the inlet temperature of the fresh catalyst bed or the regenerated catalyst bed, N represents that the switched-in fresh catalyst bed or regenerated catalyst bed is located at the logically N-th stage in the catalyst beds connected in series, $T_i^{outlet}$ represents the outlet temperature of the catalyst bed at the logically i-th stage in the catalyst beds connected in series after the fresh catalyst bed or the regenerated catalyst bed is switched in, $T_i^{inlet}$ represents the inlet temperature of the catalyst bed at the logically i-th stage, $\sum_{i=1}^{N-1}(T_i^{outlet} - T_i^{inlet})$ represents the sum of the temperature rises of all catalyst beds upstreaming the switched-in fresh catalyst bed or regenerated catalyst bed, $a_1$ represents the molar ratio of the olefin to the alkylbenzene hydroperoxide in the feed, and $b_1$ represents the molar ratio of the alkylbenzene to the alkylbenzene hydroperoxide in the feed.

2. A method for controlling the temperature of the discharge leaving a switched-in fresh catalyst bed or regenerated catalyst bed in a method for preparing alkylene oxide to be lower than or equal to 130 °C, the method including feeding a feed comprising an olefin, an alkylbenzene hydroperoxide and an alkylbenzene into an epoxidation reaction zone comprising at least two catalyst beds connected in series to carry out an epoxidation reaction to obtain a reaction product comprising an alkylene oxide, wherein the feed enters the logically first stage catalyst bed of the at least two catalyst beds connected in series and passes sequentially through the at least two catalyst beds connected in series, wherein when the logically first stage catalyst bed is deactivated, it is switched out and a fresh catalyst bed or a regenerated catalyst bed is switched in; wherein the method comprises making the inlet temperature of the feed entering the switched-in fresh catalyst bed or regenerated catalyst bed satisfy the following formula (1):

   $$40°C \leq T_N^{inlet} \leq 130°C + \sum_{i=1}^{N-1}(T_i^{outlet} - T_i^{inlet}) - \frac{1370}{a_1 + 1.38b_1} \qquad (1);$$

   in formula (1), $T_N^{inlet}$ represents the inlet temperature of the fresh catalyst bed or the regenerated catalyst bed, N represents that the switched-in fresh catalyst bed or regenerated catalyst bed is located at the logically N-th stage in the catalyst beds connected in series, $T_i^{outlet}$ represents the outlet temperature of the catalyst bed at the logically i-th stage in the catalyst beds connected in series after the fresh catalyst bed or the regenerated catalyst bed is switched in, $T_i^{inlet}$ represents the inlet temperature of the catalyst bed at the logically i-th stage, $\sum_{i=1}^{N-1}(T_i^{outlet}-T_i^{inlet})$ represents the sum of the temperature rises of all catalyst beds upstreaming the switched-in fresh catalyst bed or regenerated catalyst bed, $a_1$ represents the molar ratio of the olefin to the alkylbenzene hydroperoxide in the feed, and $b_1$ represents the molar ratio of the alkylbenzene to the alkylbenzene hydroperoxide in the feed.

3. The method according to claim 1 or 2, **characterized in that** the inlet temperature of the feed entering the switched-in fresh catalyst bed or regenerated catalyst bed satisfies the following formula (1a):

$$90°C+\sum_{i=1}^{N-1}(T_i^{outlet}-T_i^{inlet})-\frac{1370}{a_1+1.38b_1}\le T_N^{inlet}\le130°C+\sum_{i=1}^{N-1}(T_i^{outlet}-T_i^{inlet})-\frac{1370}{a_1+1.38b_1}\qquad(1a);$$

preferably, satisfies the following formula (1b):

$$100°C+\sum_{i=1}^{N-1}(T_i^{outlet}-T_i^{inlet})-\frac{1370}{a_1+1.38b_1}\le T_N^{inlet}\le130°C+\sum_{i=1}^{N-1}(T_i^{outlet}-T_i^{inlet})-\frac{1370}{a_1+1.38b_1}\qquad(1b);$$

preferably, satisfies the following formula (1c):

$$110°C+\sum_{i=1}^{N-1}(T_i^{outlet}-T_i^{inlet})-\frac{1370}{a_1+1.38b_1}\le T_N^{inlet}\le130°C+\sum_{i=1}^{N-1}(T_i^{outlet}-T_i^{inlet})-\frac{1370}{a_1+1.38b_1}\qquad(1c);$$

more preferably, the inlet temperature of the feed entering the switched-in fresh catalyst bed or regenerated catalyst bed satisfies the following formula (2):

$$120°C+\sum_{i=1}^{N-1}(T_i^{outlet}-T_i^{inlet})-\frac{1370}{a_1+1.38b_1}\le T_N^{inlet}\le130°C+\sum_{i=1}^{N-1}(T_i^{outlet}-T_i^{inlet})-\frac{1370}{a_1+1.38b_1}\qquad(2);$$

in formulae (1a), (1b),(1c) and (2), $T_N^{inlet}$ , $T_i^{outlet}$ , $T_i^{inlet}$ , $\sum_{i=1}^{N-1}(T_i^{outlet}-T_i^{inlet})$ , $a_1$ and $b_1$ are as defined in formula (1).

4. The method according to any one of claims 1 to 3, **characterized in that** the switched-in fresh catalyst bed or regenerated catalyst bed is located at logically N-th stage in the catalyst beds connected in series, wherein the sum of the temperature rises of the N-1 catalyst beds upstreaming it satisfies the following formula (3):

$$\sum_{i=1}^{N-1}(T_i^{outlet}-T_i^{inlet})>\frac{1370}{a_1+1.38b_1}-90\qquad(3);$$

preferably, satisfies the following formula (4):

$$\sum_{i=1}^{N-1}(T_i^{outlet}-T_i^{inlet})>\frac{1370}{a_1+1.38b_1}-50\qquad(4);$$

in formulae (3) and (4), N, $T_i^{outlet}$ , $T_i^{inlet}$ , $\sum_{i=1}^{N-1}(T_i^{outlet}-T_i^{inlet})$ , $a_1$ and $b_1$ are as defined in formula (1).

5. The method according to any one of claims 1 to 4, **characterized in that** the switched-in fresh catalyst bed or regenerated catalyst bed is located at the logically last stage of the catalyst beds connected in series.

6. The method according to any one of claims 1 to 5, **characterized in that** the epoxidation reaction zone comprises 2 to 20, preferably 3 to 20, preferably 3 to 10, more preferably 3 to 7 catalyst beds connected in series; and/or, the catalyst beds in the epoxidation reaction zone are loaded with an epoxidation catalyst; preferably, the epoxidation catalyst is a titanium-containing catalyst, more preferably at least one selected from titanium silicon molecular sieve, titanium dioxide and a combination thereof.

7. The method according to any one of claims 1 to 6, **characterized in that**

   the alkylene oxide is selected from propylene oxide, ethylene oxide and butylene oxide, preferably propylene oxide; and/or,
   the olefin is selected from ethylene, propylene and butene, more preferably propylene, and/or
   the alkylbenzene hydroperoxide is cumene hydroperoxide; and/or,
   the alkylbenzene is at least one selected from ethylbenzene, cumene, butylbenzene or a combination thereof, preferably cumene; and/or,
   the molar ratio of the olefin to the alkylbenzene hydroperoxide is 2 to 50, preferably 2 to 12; and/or,
   the alkylbenzene hydroperoxide is fed in the form of a solution, wherein the solution contains the alkylbenzene hydroperoxide and an alkylbenzene; preferably, the alkylbenzene is at least one selected from ethylbenzene, cumene and butylbenzene; more preferably, the concentration of alkylbenzene in the solution is 5wt% to 95wt%.

8. The method according to any one of claims 1 to 7, **characterized in that**, based on the total amount of catalyst, the mass space velocity of the alkylbenzene hydroperoxide is 0.1 to 3 $h^{-1}$, preferably 0.2 to 2 $h^{-1}$; and/or, the temperature of the epoxidation reaction is 0-200 °C, and the pressure is 2-20 MPag; preferably, the temperature of the epoxidation reaction is 40-130 °C, and the pressure is 3-12 MPag.

9. A system for preparing alkylene oxide, the system comprising an epoxidation reaction zone comprising at least two catalyst beds connected in series, and a temperature controller;

   wherein a feed comprising an olefin, an alkylbenzene hydroperoxide and an alkylbenzene enters the epoxidation reaction zone to carry out an epoxidation reaction to obtain a product comprising an alkylene oxide, wherein the feed enters the logically first stage catalyst bed of the at least two catalyst beds connected in series and passes sequentially through the at least two catalyst beds connected in series,
   wherein, the temperature controller controls the inlet temperature of the feed of the newly switched-in fresh catalyst bed or regenerated catalyst bed such that the inlet temperature satisfies the following formula (1):

$$40°C \leq T_N^{inlet} \leq 130°C + \sum_{i=1}^{N-1}(T_i^{outlet} - T_i^{inlet}) - \frac{1370}{a_1 + 1.38b_1} \quad (1);$$

   in formula (1), $T_N^{inlet}$ represents the inlet temperature of the fresh catalyst bed or the regenerated catalyst bed, N represents that the switched-in fresh catalyst bed or regenerated catalyst bed is located at the logically N-th stage in the catalyst beds connected in series, $T_i^{outlet}$ represents the outlet temperature of the catalyst bed at the logically i-th stage in the catalyst beds connected in series after the fresh catalyst bed or the regenerated catalyst bed is switched in, $T_i^{inlet}$ the inlet temperature of the catalyst bed at the logically i-th stage, $\sum_{i=1}^{N-1}(T_i^{outlet} - T_i^{inlet})$ represents the sum of the temperature rises of all catalyst beds upstreaming the switched-in fresh catalyst bed or regenerated catalyst bed, $a_1$ represents the molar ratio of the olefin to the alkylbenzene hydroperoxide in the feed, and $b_1$ represents the molar ratio of the alkylbenzene to the alkylbenzene hydroperoxide in the feed.

10. The system according to claim 9, **characterized in that** the temperature controller controls the inlet temperature of the feed to the switched-in fresh catalyst bed or regenerated catalyst bed such that the inlet temperature satisfies the following formula (1a):

$$90^{\circ}\text{C}+\sum_{i=1}^{N-1}(T_i^{outlet}-T_i^{inlet})-\frac{1370}{a_1+1.38b_1}\leq T_N^{inlet}\leq 130^{\circ}\text{C}+\sum_{i=1}^{N-1}(T_i^{outlet}-T_i^{inlet})-\frac{1370}{a_1+1.38b_1} \qquad (1a)$$

preferably, satisfies the following formula (1b):

$$100^{\circ}\text{C}+\sum_{i=1}^{N-1}(T_i^{outlet}-T_i^{inlet})-\frac{1370}{a_1+1.38b_1}\leq T_N^{inlet}\leq 130^{\circ}\text{C}+\sum_{i=1}^{N-1}(T_i^{outlet}-T_i^{inlet})-\frac{1370}{a_1+1.38b_1} \qquad (1b);$$

preferably, satisfies the following formula (1c):

$$110^{\circ}\text{C}+\sum_{i=1}^{N-1}(T_i^{outlet}-T_i^{inlet})-\frac{1370}{a_1+1.38b_1}\leq T_N^{inlet}\leq 130^{\circ}\text{C}+\sum_{i=1}^{N-1}(T_i^{outlet}-T_i^{inlet})-\frac{1370}{a_1+1.38b_1} \qquad (1c);$$

more preferably, the temperature controller controls the inlet temperature of the feed to the switched-in fresh catalyst bed or regenerated catalyst bed such that the inlet temperature satisfies the following formula (2):

$$120^{\circ}\text{C}+\sum_{i=1}^{N-1}(T_i^{outlet}-T_i^{inlet})-\frac{1370}{a_1+1.38b_1}\leq T_N^{inlet}\leq 130^{\circ}\text{C}+\sum_{i=1}^{N-1}(T_i^{outlet}-T_i^{inlet})-\frac{1370}{a_1+1.38b_1} \qquad (2);$$

in formulae (1a), (1b), (1c) and (2), $T_N^{inlet}$, N, $T_i^{outlet}$, $T_i^{inlet}$, $\sum_{i=1}^{N-1}(T_i^{outlet}-T_i^{inlet})$ $a_1$ and $b_1$ are as defined in formula (1).

11. The system according to claim 9 or 10, **characterized in that** the switched-in fresh catalyst bed or regenerated catalyst bed is located at logically N-th stage in the catalyst beds connected in series, wherein the sum of the temperature rises of the N-1 catalyst beds upstreaming it satisfies the following formula (3):

$$\sum_{i=1}^{N-1}(T_i^{outlet}-T_i^{inlet})>\frac{1370}{a_1+1.38b_1}-90 \qquad (3);$$

preferably, satisfies the following formula (4):

$$\sum_{i=1}^{N-1}(T_i^{outlet}-T_i^{inlet})>\frac{1370}{a_1+1.38b_1}-50 \qquad (4);$$

in formulae (3) and (4), N, $T_i^{outlet}$, $T_i^{inlet}$, $\sum_{i=1}^{N-1}(T_i^{outlet}-T_i^{inlet})$, $a_1$ and $b_1$ are as defined in formula (1).

12. The system according to any one of claims 9 to 11, **characterized in that**:

an olefin feed pipeline and an alkylbenzene hydroperoxide feed pipeline connected to the first catalyst bed are arranged before the at least two catalyst beds connected in series; and/or, a preheating element is arranged on the olefin feed pipeline; preferably, the preheating element is a heater or a heat exchanger; and/or, a cooling element is arranged on the connecting pipeline between two adjacent catalyst beds.

13. The system according to any one of claims 9 to 12, **characterized in that** the epoxidation reaction zone comprises 2 to 20, preferably 3 to 20, preferably 3 to 10, preferably 3 to 7, more preferably 3 to 6 catalyst beds connected in series; and/or,

the switched-in fresh catalyst bed or regenerated catalyst bed is located at the logically last stage of the catalyst beds connected in series; and/or,

the catalyst beds in the epoxidation reaction zone are loaded with an epoxidation catalyst; preferably, the epoxidation catalyst is a titanium-containing catalyst, more preferably at least one selected from titanium silicon molecular sieve, titanium dioxide and a combination thereof.

14. The system according to any one of claims 9 to 13, **characterized in that**

the inlet and outlet of each catalyst bed are each independently provided with a switching valve for switching in or switching out of catalyst bed; and/or,

the system further comprises one or more fresh catalyst bed(s) or regenerated catalyst bed(s) to be switched in; wherein a catalyst bed in the epoxidation reaction zone, preferably the logically first stage catalyst bed, is switched out after deactivation, and the fresh catalyst bed or regenerated catalyst bed is switched in the epoxidation reaction zone after the deactivated catalyst bed is switched out.

15. The system according to any one of claims 9 to 14, **characterized in that** the inlet and outlet of each catalyst bed are each independently provided with a temperature detector; and/or,

the temperature controller controls the inlet temperature of the feed to the switched-in fresh catalyst bed or regenerated catalyst bed through a cooling element; preferably, the temperature controller controls the inlet temperature of the feed to the switched-in fresh catalyst bed or regenerated catalyst bed through a cooling element arranged on the connecting pipeline between two adjacent catalyst beds; more preferably, the cooling element is electrically connected to the temperature controller.

16. The system according to any one of claims 9 to 15, **characterized in that**

the alkylene oxide is selected from propylene oxide, ethylene oxide and butylene oxide, preferably propylene oxide; and/or,

the olefin is selected from ethylene, propylene and butene, more preferably propylene, and/or

the alkylbenzene hydroperoxide is cumene hydroperoxide; and/or,

the alkylbenzene is at least one selected from ethylbenzene, cumene, butylbenzene or a combination thereof, preferably cumene; and/or,

the molar ratio of the olefin to the alkylbenzene hydroperoxide is 2 to 50, preferably 2 to 12; and/or,

the alkylbenzene hydroperoxide is fed in the form of a solution, and the solution comprises the alkylbenzene hydroperoxide and an alkylbenzene; preferably, the alkylbenzene is at least one selected from ethylbenzene, cumene and butylbenzene; more preferably, the concentration of alkylbenzene in the solution is 5wt% to 95wt%.

17. The system according to any one of claims 9 to 16, **characterized in that**, based on the total amount of catalyst, the mass space velocity of the alkylbenzene hydroperoxide is 0.1 to 3 $h^{-1}$, preferably 0.2 to 2 $h^{-1}$; and/or,

the temperature of the epoxidation reaction is 0-200 °C, and the pressure is 2-20 MPag; preferably, the temperature of the epoxidation reaction is 40-130 °C, and the pressure is 3-12 MPag.

18. The system according to any one of claims 9-17, **characterized in that** the system is used to carry out the method according to any one of claims 1-8.

Fig. 1

Fig. 2

Fig. 3

## N=6

Fig. 4

## N=5

Fig. 5

## INTERNATIONAL SEARCH REPORT

| International application No. |
| --- |
| **PCT/CN2023/127003** |

| A. | CLASSIFICATION OF SUBJECT MATTER |
| --- | --- |

C07D301/19(2006.01)i; C07D303/04(2006.01)i; B01J8/04(2006.01)i

According to International Patent Classification (IPC) or to both national classification and IPC

| B. | FIELDS SEARCHED |
| --- | --- |

Minimum documentation searched (classification system followed by classification symbols)

IPC:C07D,B01J

Documentation searched other than minimum documentation to the extent that such documents are included in the fields searched

Electronic data base consulted during the international search (name of data base and, where practicable, search terms used)

CJFD, CNTXT, ENTXTC, ENTXT, DWPI, CNKI: 氢过氧化, 过氧化氢, 过氧化氢烷基苯, 烯烃, 丙烯, 环氧化, 入口温度, 出口温度, 过氧化, 控制, 冷却, 反应热, hydroperoxide, olefin, propylene, epoxidation, inlet, outlet, temperature, control+, cool +, heat

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | US 2003153775 A1 (SUMITOMO CHEMICAL CO., LTD. et al.) 14 August 2003 (2003-08-14) description, paragraphs 4-8 and 23 | 1-18 |
| A | CN 1809546 A (SHELL INTERNATIONALE RESEARCH MAATSCHAPPIJ B.V.) 26 July 2006 (2006-07-26) description, page 2, paragraph 5, and page 5, paragraph 5 | 1-18 |
| A | US 2003097009 A1 (SUMITOMO CHEMICAL CO., LTD. et al.) 22 May 2003 (2003-05-22) entire document | 1-18 |
| A | CN 109476621 A (SUMITOMO CHEMICAL CO., LTD.) 15 March 2019 (2019-03-15) entire document | 1-18 |
| A | CN 104557782 A (CHINA PETROCHEMICAL CO., LTD. et al.) 29 April 2015 (2015-04-29) entire document | 1-18 |
| A | CN 105272938 A (CHINA PETROCHEMICAL CO., LTD. et al.) 27 January 2016 (2016-01-27) entire document | 1-18 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **22 November 2023** | **08 January 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| :--- |
| **PCT/CN2023/127003** |

**C.    DOCUMENTS CONSIDERED TO BE RELEVANT**

| Category* | Citation of document, with indication, where appropriate, of the relevant passages | Relevant to claim No. |
| :---: | :--- | :---: |
| A | US 6365761 B1 (SHELL OIL COMPANY) 02 April 2002 (2002-04-02)<br>entire document | 1-18 |
| A | US 6583300 B1 (ARCO CHEMICAL TECHNOLOGY, L.P.) 24 June 2003 (2003-06-24)<br>entire document | 1-18 |
| A | CN 104557779 A (CHINA PETROCHEMICAL CO., LTD. et al.) 29 April 2015 (2015-04-29)<br>entire document | 1-18 |
| A | CN 105272948 A (CHINA PETROCHEMICAL CO., LTD. et al.) 27 January 2016<br>(2016-01-27)<br>entire document | 1-18 |
| A | CN 112851602 A (CHINA PETROCHEMICAL CO., LTD. et al.) 28 May 2021 (2021-05-28)<br>entire document | 1-18 |

Form PCT/ISA/210 (second sheet) (July 2022)

**INTERNATIONAL SEARCH REPORT**
Information on patent family members

International application No.

**PCT/CN2023/127003**

| Patent document cited in search report | | | Publication date (day/month/year) | Patent family member(s) | | | Publication date (day/month/year) |
|---|---|---|---|---|---|---|---|
| US | 2003153775 | A1 | 14 August 2003 | EP | 1266892 | A1 | 18 December 2002 |
| | | | | EP | 1266892 | A4 | 29 September 2004 |
| | | | | EP | 1266892 | B1 | 22 November 2006 |
| | | | | ES | 2275660 | T3 | 16 June 2007 |
| | | | | WO | 0170712 | A1 | 27 September 2001 |
| | | | | DE | 60124682 | D1 | 04 January 2007 |
| | | | | JP | 2001270879 | A | 02 October 2001 |
| | | | | AU | 4120601 | A | 03 October 2001 |
| | | | | CA | 2403440 | A1 | 19 September 2002 |
| | | | | CA | 2403440 | C | 18 August 2009 |
| | | | | BR | 0109333 | A | 10 June 2003 |
| | | | | US | 6646140 | B2 | 11 November 2003 |
| | | | | KR | 20030009397 | A | 29 January 2003 |
| | | | | KR | 100784119 | B1 | 12 December 2007 |
| | | | | ATE | 346054 | T1 | 15 December 2006 |
| CN | 1809546 | A | 26 July 2006 | EP | 1392668 | A1 | 03 March 2004 |
| | | | | EP | 1392668 | B1 | 29 September 2004 |
| | | | | ES | 2224067 | T3 | 01 March 2005 |
| | | | | AU | 2002302597 | B2 | 20 December 2007 |
| | | | | US | 2004210067 | A1 | 21 October 2004 |
| | | | | US | 7301038 | B2 | 27 November 2007 |
| | | | | BR | 0209445 | A | 03 August 2004 |
| | | | | WO | 02090340 | A1 | 14 November 2002 |
| | | | | ATE | 277918 | T1 | 15 October 2004 |
| | | | | DE | 60201446 | D1 | 04 November 2004 |
| | | | | DE | 60201446 | T2 | 27 October 2005 |
| | | | | JP | 2004530681 | A | 07 October 2004 |
| | | | | AU | 2002302597 | A1 | 03 March 2004 |
| US | 2003097009 | A1 | 22 May 2003 | KR | 20030009398 | A | 29 January 2003 |
| | | | | KR | 100783302 | B1 | 10 December 2007 |
| | | | | BR | 0109389 | A | 08 April 2003 |
| | | | | WO | 0170713 | A1 | 27 September 2001 |
| | | | | DE | 60124512 | D1 | 28 December 2006 |
| | | | | CA | 2403589 | A1 | 27 September 2001 |
| | | | | ATE | 345333 | T1 | 15 December 2006 |
| | | | | EP | 1266893 | A1 | 18 December 2002 |
| | | | | EP | 1266893 | A4 | 06 October 2004 |
| | | | | EP | 1266893 | B1 | 15 November 2006 |
| | | | | ES | 2274872 | T3 | 01 June 2007 |
| | | | | AU | 4120701 | A | 03 October 2001 |
| | | | | US | 6620951 | B2 | 16 September 2003 |
| | | | | JP | 2001270871 | A | 02 October 2001 |
| | | | | CN | 109476621 | B | 27 September 2022 |
| CN | 109476621 | A | 15 March 2019 | US | 2020140403 | A1 | 07 May 2020 |
| | | | | US | 10807961 | B2 | 20 October 2020 |
| | | | | KR | 20190034559 | A | 02 April 2019 |
| | | | | KR | 102407301 | B1 | 10 June 2022 |
| | | | | JPWO | 2018021271 | A1 | 09 May 2019 |
| | | | | JP | 7018880 | B2 | 14 February 2022 |
| | | | | SA | 519400978 | B1 | 13 April 2022 |

Form PCT/ISA/210 (patent family annex) (July 2022)

<table>
<tr><td colspan="2" rowspan="2">INTERNATIONAL SEARCH REPORT<br>Information on patent family members</td><td>International application No.</td></tr>
<tr><td>**PCT/CN2023/127003**</td></tr>
</table>

| Patent document cited in search report | | | Publication date (day/month/year) | Patent family member(s) | | | Publication date (day/month/year) |
|---|---|---|---|---|---|---|---|
| | | | | WO | 2018021271 | A1 | 01 February 2018 |
| | | | | RU | 2019101635 | A | 28 August 2020 |
| | | | | RU | 2019101635 | A3 | 28 October 2020 |
| | | | | RU | 2738231 | C2 | 09 December 2020 |
| | | | | EP | 3495356 | A1 | 12 June 2019 |
| | | | | EP | 3495356 | A4 | 15 January 2020 |
| | | | | EP | 3495356 | B1 | 20 September 2023 |
| | | | | EP | 3495356 | B8 | 25 October 2023 |
| | | | | MY | 189509 | A | 16 February 2022 |
| CN | 104557782 | A | 29 April 2015 | None | | | |
| CN | 105272938 | A | 27 January 2016 | None | | | |
| US | 6365761 | B1 | 02 April 2002 | BR | 0013358 | A | 30 April 2002 |
| | | | | RU | 2241706 | C2 | 10 December 2004 |
| | | | | ES | 2186655 | T3 | 16 May 2003 |
| | | | | EP | 1204653 | A1 | 15 May 2002 |
| | | | | EP | 1204653 | B1 | 29 January 2003 |
| | | | | DE | 60001325 | D1 | 06 March 2003 |
| | | | | DE | 60001325 | T2 | 04 September 2003 |
| | | | | TWI | 280959 | B | 11 May 2007 |
| | | | | AU | 6702400 | A | 13 March 2001 |
| | | | | AU | 757612 | B2 | 27 February 2003 |
| | | | | JP | 2003507374 | A | 25 February 2003 |
| | | | | WO | 0112617 | A1 | 22 February 2001 |
| US | 6583300 | B1 | 24 June 2003 | JP | 2006513155 | A | 20 April 2006 |
| | | | | JP | 4464825 | B2 | 19 May 2010 |
| | | | | BR | 0315277 | A | 30 August 2005 |
| | | | | KR | 20050070061 | A | 05 July 2005 |
| | | | | KR | 100987066 | B1 | 11 October 2010 |
| | | | | DE | 60303830 | D1 | 27 April 2006 |
| | | | | DE | 60303830 | T2 | 26 October 2006 |
| | | | | AU | 2003260134 | A1 | 04 May 2004 |
| | | | | CA | 2502509 | A1 | 29 April 2004 |
| | | | | ATE | 318802 | T1 | 15 March 2006 |
| | | | | ES | 2254987 | T3 | 16 June 2006 |
| | | | | EP | 1551819 | A1 | 13 July 2005 |
| | | | | EP | 1551819 | B1 | 01 March 2006 |
| | | | | WO | 2004035559 | A1 | 29 April 2004 |
| CN | 104557779 | A | 29 April 2015 | None | | | |
| CN | 105272948 | A | 27 January 2016 | CN | 105272948 | B | 06 April 2018 |
| CN | 112851602 | A | 28 May 2021 | None | | | |

Form PCT/ISA/210 (patent family annex) (July 2022)

**EP 4 610 255 A1**

**Patent documents cited in the description**

- CN 1692106 A
- CN 105294606 A
- CN 110787739 A
- CN 1325484 C
- CN 105272947 A
- CN 104437618 B